# EUROPEAN PATENT SPECIFICATION

(11) **EP 4 525 711 B1**
(45) Date of publication and mention of the grant of the patent: **22.04.2026**
(21) Application number: 23731838.1
(22) Date of filing: 18.05.2023
(51) Int. Cl.: A61B 5/11, A61B 5/00

(54) **MACHINE LEARNING ACTIVATED GAIT ASSISTANCE**
DURCH MASCHINENLERNEN AKTIVIERTE GANGHILFE
AIDE À LA MARCHE ACTIVÉE PAR APPRENTISSAGE AUTOMATIQUE

(30) Priority: 19.05.2022 US 202263364973 P; 08.12.2022 US 202263386646 P
(43) Date of publication of application: 26.03.2025
(73) Proprietor: THE CENTER FOR HEADACHE, SPINE, AND PAIN MEDICINE, PLLC, Dallas, Texas 75230 (US)
(72) Inventor: FINK, Ezekiel, Dallas, Texas 75230 (US); BROWDER, Krag, Colleyville, Texas 76034 (US); HAMBURGER, Adrian, Sarasota, Florida 34238 (US); GILLETTE, Conner, Irving, Texas 75060 (US)
(74) Representative: Schwarz & Partner Patentanwälte GmbH
(86) International application number: PCT/US2023/022775
(87) International publication number: WO 2023/225224

(56) References cited:
- WO-A1-2021/068038
- WO-A1-2022/013678
- US-A1- 2020 093 400
- US-A1- 2021 248 215
- KAISSIS GEORGIOS A. ET AL: "Secure, privacy-preserving and federated machine learning in medical imaging", NATURE MACHINE INTELLIGENCE, vol. 2, no. 6, 1 June 2020 (2020-06-01), pages 305 - 311, XP055920291, Retrieved from the Internet <URL:https://www.nature.com/articles/s42256-020-0186-1.pdf> DOI: 10.1038/s42256-020-0186-1

## Description

### CROSS-REFERENCE TO RELATED APPLICATIONS

This application claims the benefit of U.S. Provisional Application Serial No. 63/364,973, titled "Machine Learning Activated Gait Assistance," filed by Ezekiel Fink, et al., on May 19, 2022, and the benefit of U.S. Provisional Application Serial No. 63/386,646, titled "Machine Learning Activated Gait Assistance," filed by Ezekiel Fink, et al., on December 8, 2022.

### TECHNICAL FIELD

Various embodiments relate generally to systems and methods to assist patients with gait impairment and other balance impairment conditions.

### BACKGROUND

Neurological abnormal conditions may at times create difficulty in walking. Human gait, for example, may depend on a complex interplay of many parts of the nervous, cardiovascular, and musculoskeletal systems. Observing a patient walk is deemed by many one of the most important neurological examinations. In some examples, gait abnormality may be a deviation from normal walking. A safe and normal walking may require, for example, intact cognition, executive control, vision, coordination, sensory perception, motor function, autonomic function, central and peripheral nervous system mediated balance as well as other factors in combination thereof. In general, a number of issues with gait impairment or gait abnormality may increase with age. An improvement in gait impairment, as represented by reversal of a positive Romberg test, is possible using vibration stimuli in the periphery.

In some examples, a system for maintaining stability for a human may provide three inputs to the cerebellum to maintain stability: (1) vision, (2) proprioception, and (3) vestibular sense. "Proprioception," or kinesthesia, is, in general, the body's ability to sense its location, movements, and actions. The vestibular sense relates to one's sense of balance from the sensory organs (e.g., utricle, saccule, and the three semicircular canals) located near the cochlea in the inner ear. With respect to maintaining stability, only two of the three systems (1)-(3) may, for example, be needed to maintain balance.

In assessing a person's sense of balance, at times the Romberg Test is used. The Romberg Test is used for the clinical assessment of patients with disequilibrium or ataxia from sensory and motor disorders. In the test, a patient is asked to stand with her feet together and typically with her arms next to the body or crossed in front. The clinician then asks the patient to stand quietly with eyes open and, subsequently, with eyes closed. The patient tries to maintain balance with the eyes closed. If they lose balance, it is an indication that one of the other two sensory systems has an issue.

US 2020/0093400 A1 discloses systems, devices and method for the treatment of osteoarthritis.

### SUMMARY

Apparatus and associated methods relate to assisting gait impaired patients. In an illustrative example, a gait assisting apparatus may be wearable by a user including a sensor module and an actuator. The sensor module may be configured to generate a sensor measurement from measured data associated with the user. For example, a controller operably coupled to the sensor module may include a local classification model configured to classify a gait situation based on a classification input received from the sensor module. In some implementations, an activation module of the controller may generate an activation level to control the actuator. In operation, the activation module may apply the local classification model to the classification input to determine the activation level of the actuator to generate a vibration gait assistance and/or illumination guidance. Various embodiments (including auditory guidance) may advantageously provide a gait assistant function to prevent gait impairment injuries.

Apparatus and associated methods relate to a selectively activated haptic proprioception augmentation device (SAHPAD). In an illustrative example, the SAHPAD may be configured as a wearable (e.g., an anklet). The SAHPAD may, for example, apply haptic feedback (e.g., vibration) to bone structures of one or more extremities, or to tendon structures. The haptic feedback may, for example, be selectively activated as a function of sensor data corresponding to gait stability. For example, the sensor data may be received from one or more sensors of the SAHPAD. The haptic feedback may, for example, be generated in response to a command from a machine learning system (e.g., classification model) dynamically updated based on sensor data from the SAHPAD and/or other SAHPADs. Various embodiments may advantageously increase gait stability in patients with neurological deficits.

In an illustrative embodiment, a wearable vibration device may, for example, be applied to the patient's ankle to provide vibration to the skeletal system on the leg to assist with proprioception for the patient with respect to their legs. This vibration may, for example, help the patient improve stability and help reduce falls. This may, for example, be particularly helpful for a patient with peripheral neuropathy that makes them prone to fall. The vibrations may, for example, be conducted through the bone (bone conduction) or tendons, which may, for example, help the patient with stability.

Various embodiments may achieve one or more advantages. For example, some embodiments may include audio guidance to advantageously provide additional assistance to vision impaired users. Some embodiments may, for example, include preloaded classification models to advantageously improve response time of the gait assisting apparatus. For example, some embodiments may be releasably attached to a disposable coupling mechanism to advantageously allow reusability of the gait assisting apparatus across multiple patients. Some embodiments, for example, may advantageously include classification models that bias to a wearer.

The details of various embodiments are set forth in the accompanying drawings and the description below. Other features and advantages will be apparent from the description and drawings, and from the claims.

### BRIEF DESCRIPTION OF THE DRAWINGS

FIG. 1A, FIG. 1B, FIG. 1C, FIG. 1D, FIG. 1E, and FIG. 1F depict various embodiments of an exemplary Dual Platform Activated Gait Assistance (DPAGA) system employed in an illustrative use-case scenario.
FIG. 2 is an anterior view of a patient's lower right extremity.
FIG. 3 is a posterior perspective view of a patient's lower right extremity.
FIG. 4 is a plan view of an illustrative embodiment of an exemplary wearable vibration device shown on an exterior side.
FIG. 5 is a plan view of the wearable vibration device of FIG. 4 shown on an interior side.
FIG. 6 is a schematic plan view on an exterior of an illustrative embodiment of an exemplary wearable vibration device.
FIG. 7 is a schematic plan view on an interior side of the wearable vibration device of FIG. 6.
FIG. 8 is a schematic diagram of an illustrative embodiment of an exemplary circuit for use as an aspect of an illustrative embodiment of a wearable vibration device.
FIG. 9 is a plan view of an illustrative embodiment of a wearable vibration device 104 shown on an exterior side.
FIG. 10 is a plan view of an illustrative embodiment of a wearable vibration device shown on an interior side.
FIG. 11 is a block diagram of an exemplary remote controlled wearable vibration device.
FIG. 12 is a flow diagram showing an exemplary method for providing AI based gait assistance.
FIG. 13 is a flow diagram of an exemplary online training method for providing AI based gait assistance.
FIG. 14A and FIG. 14B are block diagrams of an exemplary gait assistance device including a preloaded classification model.
FIG. 15 is a flow diagram of an exemplary pre-training method for providing AI based gait assistance by an exemplary gait assistance device.
FIG. 16 is a flow diagram of an exemplary configuration method for providing AI based gait assistance by an exemplary gait assistance device.
FIG. 17 is a flow diagram of an exemplary assistance method for gait impaired users using an exemplary gait assistance device.

Like reference symbols in the various drawings indicate like elements.

### DETAILED DESCRIPTION OF ILLUSTRATIVE EMBODIMENTS

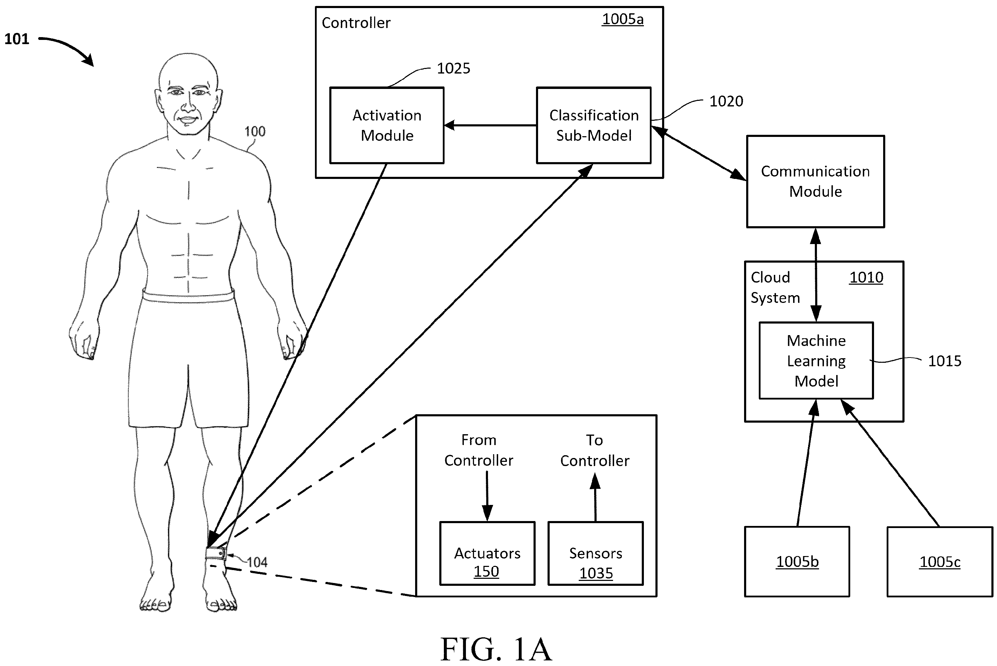

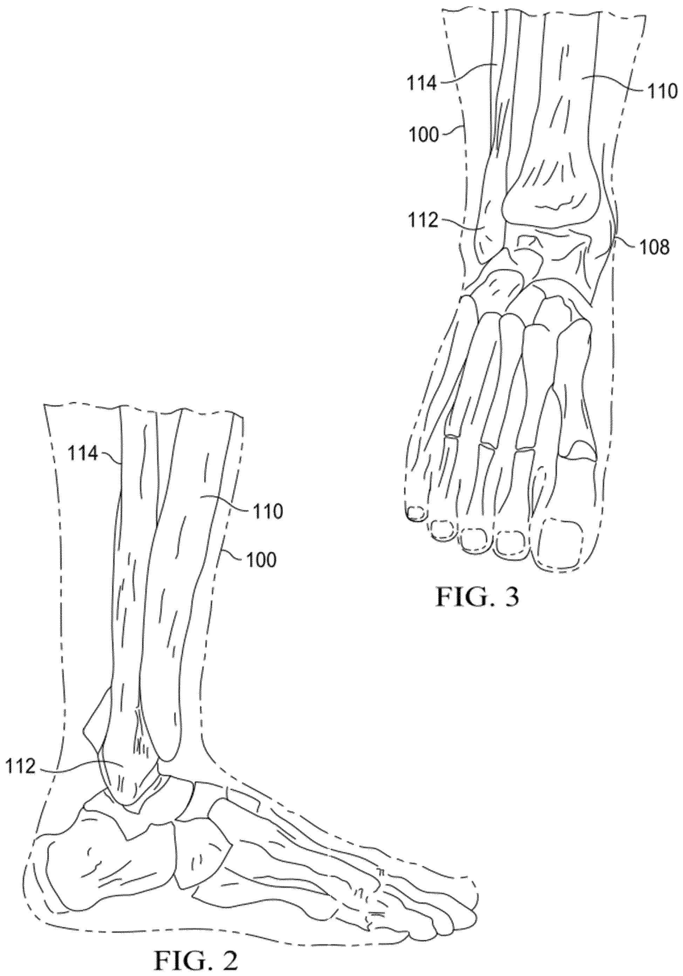

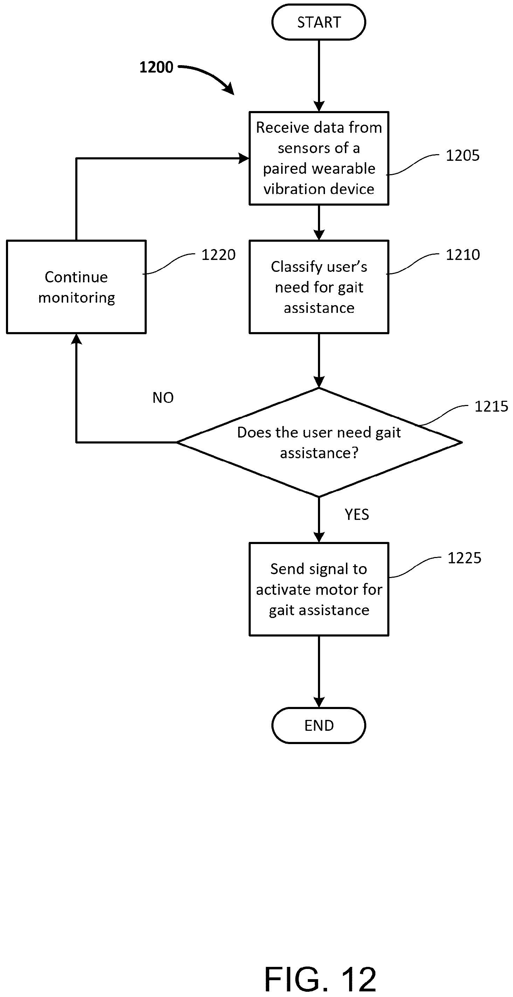

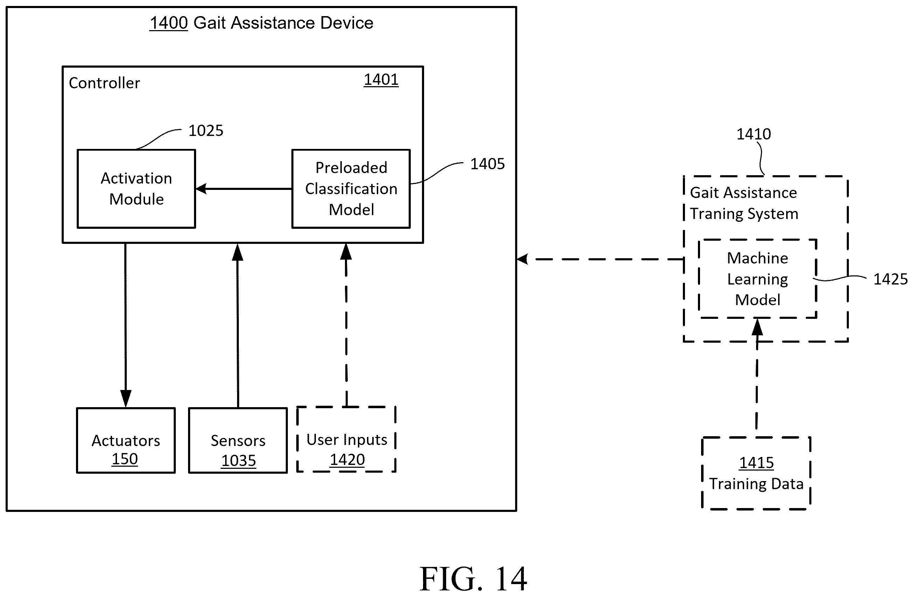

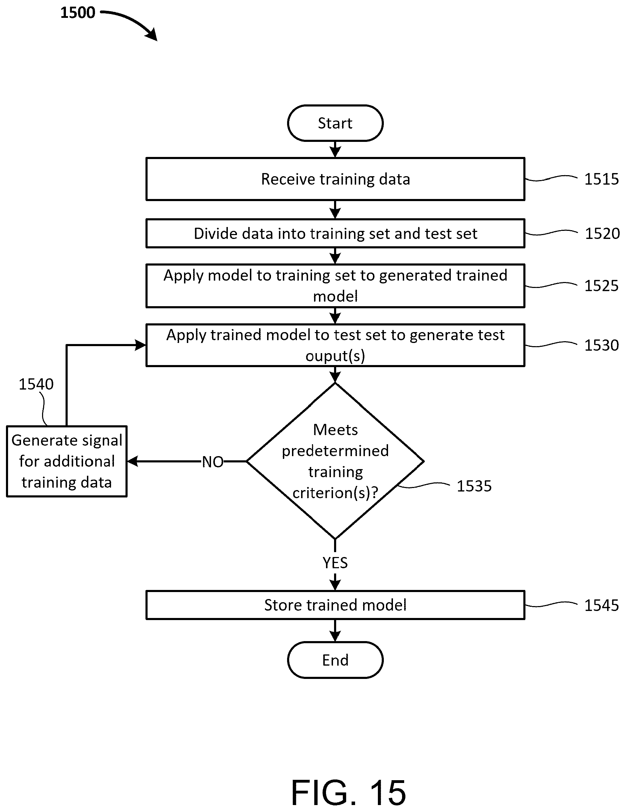

To aid understanding, this document is organized as follows. First, to help introduce discussion of various embodiments, a selectively activated haptic proprioception augmentation device (SAHPAD) is introduced with reference to FIGS. 1A-F in the context of a Dual Platform Activated Gait Assistance (DPAGA) system. Second, that introduction leads into a description with reference to FIGS. 2-11 of some exemplary embodiments of a wearable vibration device as an illustrative implementation of a SAHPAD in a DPAGA system. Third, with reference to FIGS. 12-13, illustrative methods are described in application to exemplary gait assistance and associated machine-learning training. Fourth, with reference to FIGS. 14A-B, the discussion turns to exemplary embodiments that illustrate an exemplary gait assistance device using a preloaded machine learning model. Fifth, and with reference to FIGS. 15-17, this document describes exemplary apparatus and methods useful for providing gait assistance to a gait impaired user in real time. Finally, the document discusses further embodiments, exemplary applications and aspects relating to selectively activated haptic proprioception augmentation devices and dual platform activated gait assistance systems.

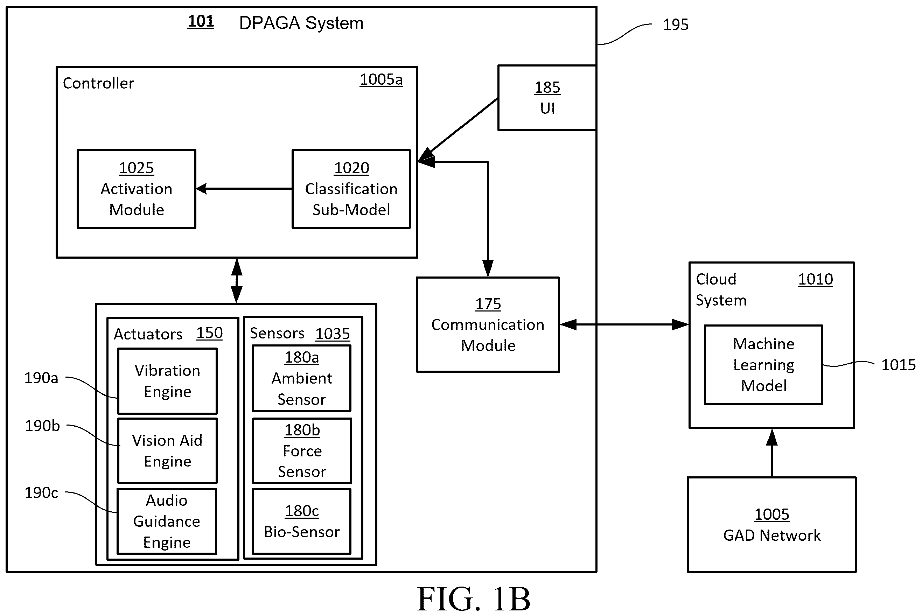

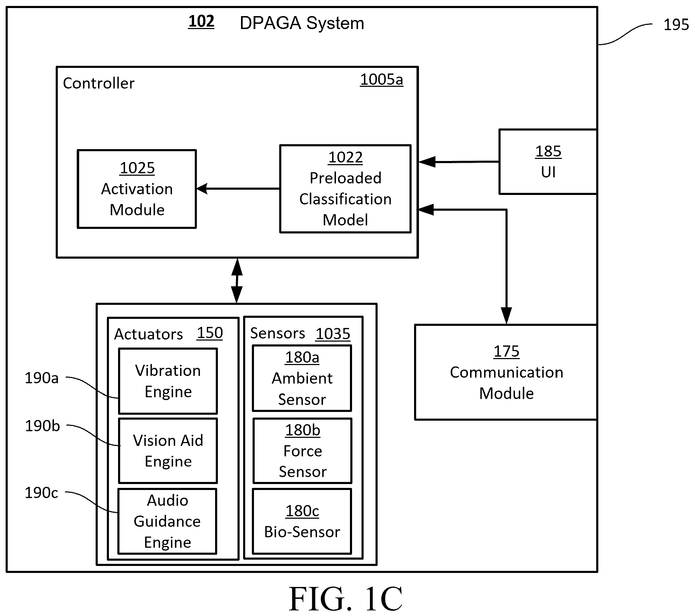

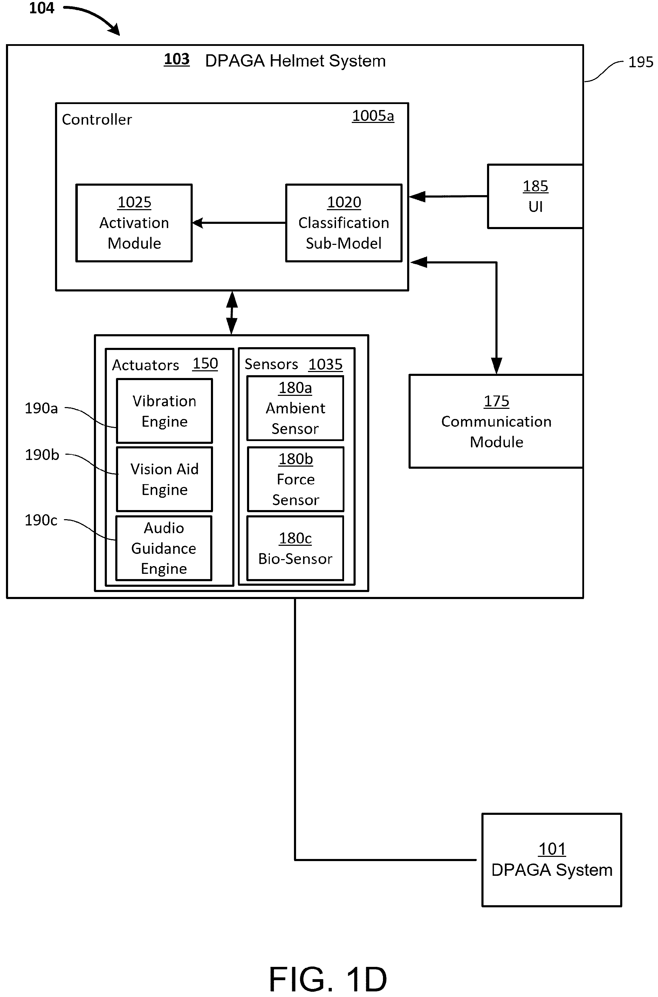

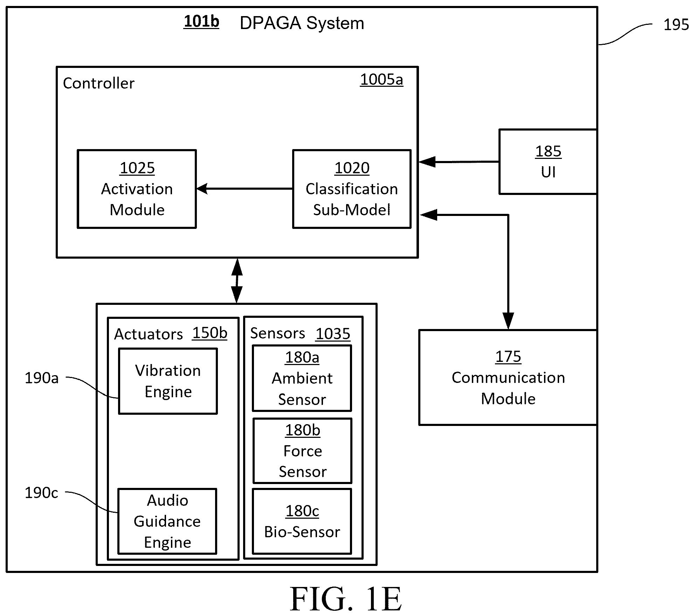

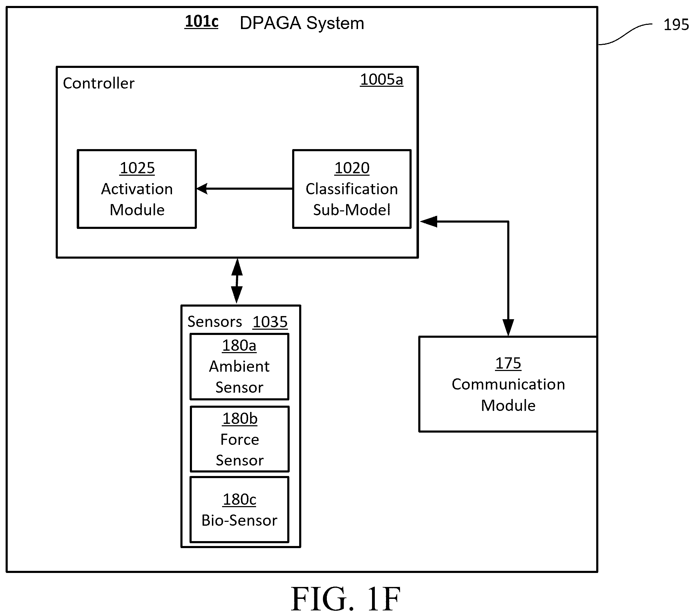

FIG. 1A, FIG. 1B, FIG. 1C, FIG. 1D, FIG. 1E, and FIG. 1F depict various embodiments of an exemplary Dual Platform Activated Gait Assistance (DPAGA) system employed in an illustrative use-case scenario. As shown in FIG. 1A, an exemplary Dual Platform Activated Gait Assistance system (DPAGA system 101) employed in an illustrative use-case scenario. In some examples, the DPAGA system 101 may be used to provide in-time assistance for a user 100 (e.g., a patient, a player, an athlete) predicted to have trouble with gait (e.g., a gait impaired patient). For example, the user 100 may have an impaired peripheral sensation. In some examples, the user 100 may have impaired vision in a dark room, causing the user 100 to lose balance due to losing two of three mechanisms for gait control. For example, the DPAGA system 101 may determine to activate a gait assisting mode based on one or more machine learning models.

In the depicted example, the user 100 is wearing a wearable vibration device 104 on a left ankle. For example, the user 100 may be wearing the wearable vibration device 104 to help with gait impairment. In some implementations, the wearable vibration device 104, when activated, may vibrate. The wearable vibration device 104 may, for example, provide haptic feedback to the user 100 through an ankle bone (e.g., medial malleolus, lateral malleolus) and/or a soft tissue (e.g., an ankle tendon) of the user 100. The vibration may, for example, assist with proprioception for the user 100 with respect to the position of their legs or feet. For example, the DPAGA system 101 may advantageously help the patient gain stability. Accordingly, the DPAGA system 101 may advantageously, for example, reduce the frequency of falls that might otherwise occur.

In this example, the wearable vibration device 104 is operably coupled to a controller 1005a. For example, the controller 1005a may be connected to the wearable vibration device 104 wirelessly through a Bluetooth connection. For example, the controller 1005a may be connected to the wearable vibration device 104 wirelessly through a wireless network (e.g., a Wireless Fidelity (WIFI) connection) connection. In some implementations, the controller 1005a may be paired with the wearable vibration device 104 to transmit and/or receive information (e.g., instructions, commands, feedback, sensor data). For example, the wearable vibration device 104 may send a signal to activate the wearable vibration device 104 to start vibration. In some implementations, the controller 1005a may classify, based on information received from the wearable vibration device 104, whether the gait assisting mode is to be activated.

The controller 1005a is, as shown, operably coupled to a cloud system 1010. For example, the controller 1005a may be connected to the cloud system 1010 via the Internet. In some implementations, the DPAGA system 101 may be an Internet of Things (IoT) system connected to the cloud system 1010. For example, the cloud system 1010 may receive information from various sensors and transmit information to various devices through the Internet based on the received information. In this example, the cloud system 1010 is further connected to controllers 1005b, 1005c.

In this example, the cloud system 1010 includes a machine learning model 1015. The machine learning model 1015 may receive input from the controllers 1005a to train, for example, a classification model for gait assistance. In some implementations, by way of example and not limitation, the machine learning model 1015 may be a weighted sum of various inputs from the controllers 1005a-c.

As depicted, the controller 1005a includes a classification sub-model 1020 and an activation module 1025. For example, the classification sub-model 1020 may include a machine learning (ML) model for classifying various gait situations for the user 100. For example, the gait situations may include that the user 100 does not need gait assistance, needs stronger gait assistance, or needs less vigorous gait assistance. For example, the classification sub-model 1020 may include an ML model configured to classify the gait situation based on time, user input, sensor input, and other data. In this example, the classification sub-model 1020 receives input from the wearable vibration device 104 and from the cloud system 1010. For example, the classification sub-model 1020 may be trained by the received input. In some implementations, the machine learning model 1015 may also be trained by data received from the classification sub-model 1020.

Based on a classification result from the classification sub-model 1020 (e.g., the gait situation), the activation module 1025 may, for example, transmit instructions to the wearable vibration device 104. For example, the activation module 1025 may turn on a vibration mode when the activation module 1025 determines that the user 100 needs gait assistance based on the classification result. For example, the activation module 1025 may increase vibration intensity when the activation module 1025 determines that the user 100 needs more gait assistance based on the classification sub-model 1020.

The wearable vibration device 104 includes one or more actuators 150 and one or more sensors 1035. For example, the actuators may include a vibration motor. The actuators 150, for example, may receive remote instruction from the activation module 1025 to activate or deactivate vibration. In some implementations, the DPAGA system 101 may continuously monitor a behavior of the user 100 and surrounding environment of the user 100. The data may be transmitted, as shown in this example, to the classification sub-model 1020. Upon classifying an action for the wearable vibration device 104, the activation module 1025 may send a signal to the wearable vibration device 104 to control the actuators 150.

In some implementations, the DPAGA system 101 may also receive user input for training the classification sub-model 1020 and the machine learning model 1015. For example, the DPAGA system 101 may receive user feedback (e.g., via a mobile device connected to the wearable vibration device 104) to indicate an unnecessary gait assistance activation. Accordingly, in some implementations, the DPAGA system 101 may advantageously actively prevent the patient from losing balance based on an adaptive AI and machine learning approach using training data from more than one patients. For example, the DPAGA system 101 may advantageously prevent injury of the user 100.

Referring to FIG. 1B, a block diagram of the DPAGA system 101 is shown. In this example, the DPAGA system 101 includes a communication module 175. For example, the communication module 175 may communicate with wearable devices of the user 100. For example, the DPAGA system 101 may receive input from other communicating wearables (e.g., smart watch, mobile devices, smart glasses) on a body of the user 100. For example, the communication module 175 may communicate to the cloud system 1010. In some implementations, the communication module 175 may be connected to the cloud system 1010 via the Internet. In some implementations, the communication module 175 may be connected to the cloud system 1010 via a local area network (e.g., via one or more routers, switch, other network devices).

The communication module 175, as shown, may transmit and receive data to and from the controller 1005a. For example, the controller 1005a may transmit the classification sub-model 1020 to the cloud system 1010 via the communication module 175.

As shown, the cloud system 1010 receives input from a gait assistance device (GAD) network 1005 (e.g., the controller 1005b and the controller 1005c). For example, the cloud system 1010 may train the machine learning model 1015 based on a classification sub-model received from a corresponding GAD in the GAD network 1005. For example, upon receiving training results from the cloud system 1010, the controller 1005a may update the classification sub-model 1020 based on information received from the communication module 175.

The controller 1005a may receive sensor data from the one or more sensors 1035. For example, the activation module 1025 may apply the sensor data to the classification sub-model 1020 to generate an activation signal to the actuators 150. In this example, the one or more sensors 1035 include an ambient sensor 180a, a force sensor 180b, and a bio-sensor 180c. The ambient sensor 180a, for example, may detect environment parameters around the DPAGA system 101. For example, the ambient sensor 180a may include a temperature sensor, a distance sensor, and an ambient light sensor. For example, the activation module 1025 may activate an illumination guidance when the ambient light level is low. For example, the distance sensor may include a time-of-flight sensor configured to measure a distance to floor. For example, the distance sensor may include an ultrasonic sensor. For example, the distance sensor may include an infra-red sensor. For example, the controller 1005a may generate an alert based on distance measurement to advantageously prevent collisions of the user 100 to another object.

For example, multiple time-of-flight sensors (e.g., 2, 3, 6) may be used. In some implementations, one or more of the multiple distance sensors may be switched off based on an orientation of the wearable vibration device 104. For example, two distance sensors (e.g., one facing forward and one facing downwards for gait analysis) may be activated for measurement. For example, the 3^{rd} distance sensor may be redundant. For example, when the wearable vibration device 104 is placed reversely (e.g., upside down), the controller 1005a may recognize that and calibrate the time-of-flight sensors for accurate data capture.

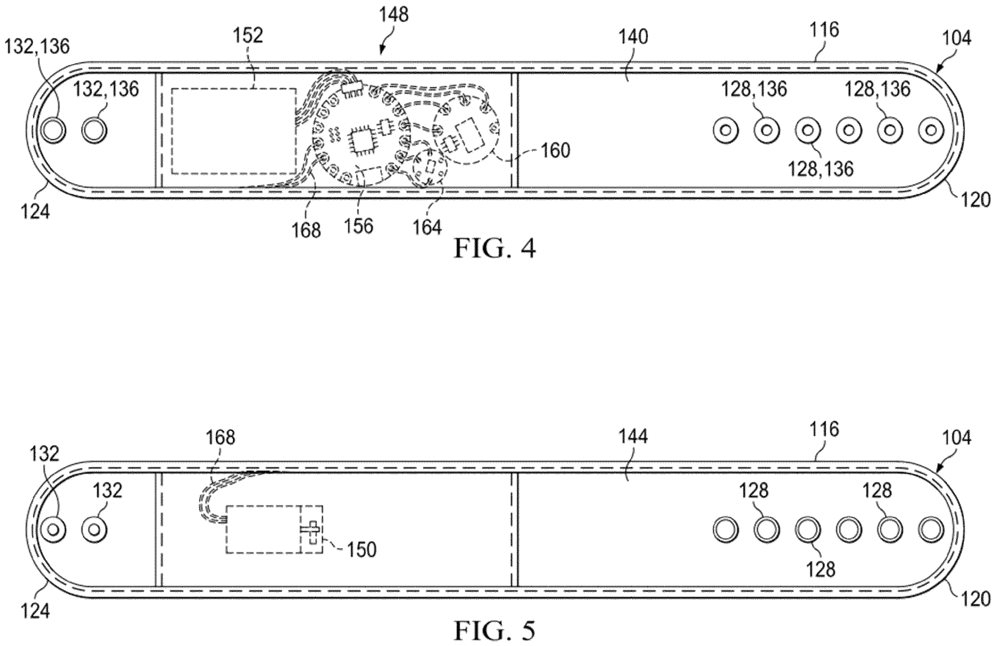

The force sensor 180b, for example, may detect a relative position of the wearable vibration device to the user during limb movements. For example, the controller 1005a may determine whether and/or how the user 100 is moving based on data received from the force sensor 180b. The bio-sensor 180c may, for example, detect a health-related situation of the user 100. For example, the controller 1005a may determine whether the user 100 is too fatigued. For example, the activation module 1025 may advantageously provide audio suggest/guidance to the user 100 based on the health information. Various embodiments of the sensors 1035 are further described with reference to FIG. 5.

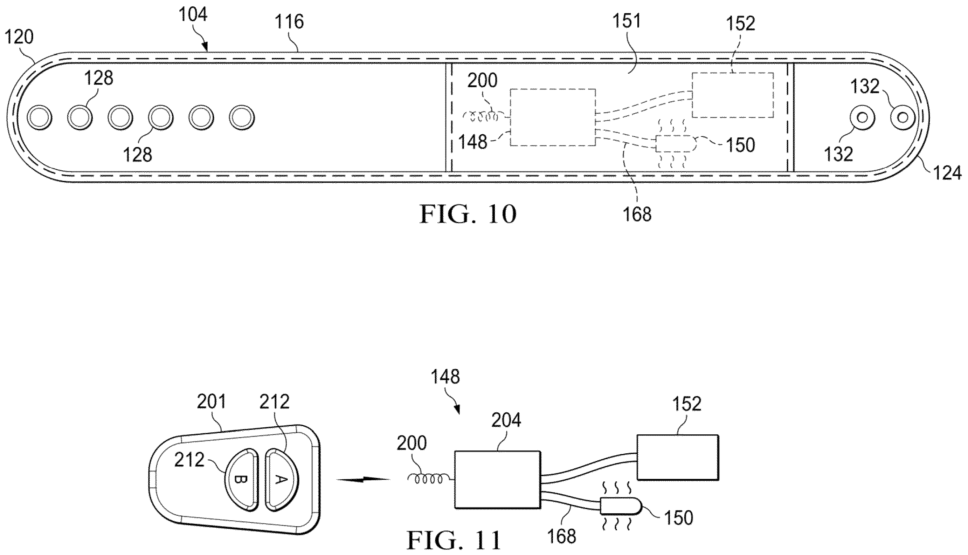

The DPAGA system 101 also includes a user interface 185. For example, the user 100 may activate a monitor mode of the DPAGA system 101 using the user interface 185. In some implementations, the user interface 185 may be disposed on a housing 195 of the DPAGA system 101. For example, the user interface 185 may be an on/off switch. For example, the user interface 185 may be a turning knob. In some implementations, the user interface 185 may be connected to the controller 1005a remotely. For example, the user interface 185 may be a remote control device. Some exemplary embodiments of the user interface 185 may be described with reference to FIG. 11.

In some implementations, the user may use the user interface 185 to control a mode of operations for the DPAGA system 101. For example, the activation module 1025 may generate an activation signal based on received user input. For example, the user may set a maximum vibration allowed for the actuators 150. For example, the user may set a minimum intensity for the actuators 150 for effective gait assistance. In some implementations, the user interface 185 may control a type of gait assistance (e.g., motion assistance, voice guidance, audio guidance) is provided when the DPAGA system 101 is activated.

As shown, the actuators 150 includes a vibration engine 190a, a vision aid engine 190b, and an audio guidance engine 190c. For example, the vibration engine 190a may include a vibration motor. For example, the vision aid engine 190b may include a light emitting diode (LED) module. For example, the activation module 1025 may selectively activate the LED module to illuminate a path ahead of the user 100 to provide illumination guidance. The audio guidance engine 190c, for example, may include a buzzer. For example, the audio guidance engine 190c may increase an intensity of a beeping sound when the user 100 is getting closer to an obstacle, and/ or as an auditory cue for imminent or actual foot contact with floor. In some implementations, the audio guidance engine 190c may include a speaker. For example, the audio guidance engine 190c may, once activated, provide audio guidance to the user 100 based on the gait situation classified by the classification sub-model 1020.

In some implementations, the audio guidance engine 190c may generate an auditory signal triggered by the activation module 1025. For example, the activation module 1025 may determine that a warning to the user 100 for imminent fall is to be generated after gait analysis. In some implementations, the audio guidance engine 190c may generate a warning for fall that has happened. For example, the warning may notify a helper to assist a fallen user. In some implementations, the audio guidance engine 190c may generate an auditory guide to assist a visually impaired user to navigate a space.

In various implementations, the user 100 may be configured to connect to a remote federated machine learning model (e.g., the machine learning model 1015). For example, the DPAGA system 101 may include a local learning model and sensors. In some implementations, the federated machine learning model may be connected to a network of remote GADs (e.g., the GAD network 1005) in data communication with the federated machine learning model.

For example, the DPAGA system 101 may, in an online mode, receive update weighting (e.g., via the communication module 175 from the federated machine learning model) as a function of training inputs from the network of remote GADs. For example, the training inputs may include updated training output parameters generated by local learning models of the remote GADs based on corresponding GAD sensor inputs (e.g., from the one or more sensors 1035 of each of the remote GADs). In an offline mode, the DPAGA system 101 may independently select to activate, as a function of the local learning model (e.g., the classification sub-model 1020) in response to the sensor inputs. Accordingly, the DPAGA system 101 may advantageously provide a gait assistant function to a wearer of the DPAGA system 101. In some examples, because the machine learning model 1015 receives input from the 1020 of the DPAGA system 101, a resulting classification sub-model 1020 may be biased to the user 100 (e.g., to local environment situations, to own health conditions).

In some implementations, the DPAGA system 101 may also include a vision assistance module (e.g., the vision aid engine 190b) configured to produce an illumination guidance for gait impaired users. For example, the vision assistance module may be activated as a function of the local learning model (e.g., the classification sub-model 1020). Accordingly, for example, the DPAGA system 101 may advantageously provide pre-injury detection and/or prevention in real time. For example, the DPAGA system 101 may be configured as a pre-injury detection and/or prevention system.

FIG. 1C shows an exemplary DPAGA system 102. As shown, the DPAGA system 102 includes the user interface 185, the actuators 150, and the one or more sensors 1035. In this example, the controller 1005a includes a preloaded classification model 1022. For example, the 1022 may be centrally trained before installed into the DPAGA system 102 (e.g., during a manufacturing process). In some implementations, a user 100 may update (e.g., upgrade) the [no text] 1022 using the user interface 185.

A DPAGA Helmet system 103 is shown in FIG. 1D. In some implementations, the wearable vibration device 104 may be worn in other location of the patient's body. For example, the wearable vibration device 104 may be worn on an upper extremity, lower extremity, trunk, head, neck, genitalia and/or orifices (mouth, anus, introitus)of the user 100. In some implementations, the wearable vibration device 104 may be configured to be worn on the head of the user 100 (e.g. as DPAGA Helmet system 103).

In some implementations, for example, a pre-injury detection system may include a wearable vibration device (e.g., the wearable vibration device 104) coupled to an upper extremity of a user (e.g., a user's head such as by a helmet and/or other headwear) and coupled to a lower extremity of a user (e.g., a leg, an ankle, such as by a strap). A controller (e.g., a central controller, a remote controller) may interact with multiple of the wearable vibration devices (e.g., on the upper extremity and on the lower extremity) to detect and/or prevent injury. For example, the controller may generate a warning (e.g., haptic, auditory, visual, and/or electrical stimulus) for delivery to the user if instability is detected.

In this example, the DPAGA Helmet system 103 is operably coupled to the DPAGA system 101. For example, the DPAGA Helmet system 103 and the DPAGA system 101 may communicate to collectively prevent injury (e.g., for sports athletes during training or playing games). In some implementations, the classification sub-model 1020 of the DPAGA Helmet system 103 and the classification sub-model 1020 of the DPAGA system 101 may be specifically trained so that the actuators 150 of both systems 101, 103 may be selectively actuated to assist a stability of the user 100.

FIG. 1E shows a second embodiment of a DPAGA system 101b. For example, the DPAGA system 101b may include an actuator 150b. In this example, the 150b includes the vibration engine 190a and the audio guidance engine 190c. For example, the DPAGA system 101b may advantageously prevent from disturbing others in a dark environment by using an assistant light.

FIG. 1F shows a third embodiment of a DPAGA system 101c. In this example, the DPAGA system 101c does not include an actuator 150b. For example, the DPAGA system 101c may be used as a monitoring device. In some implementations, multiple DPAGA systems 101c may be deployed to multiple locations on a body of the user 100 (e.g., on an upper and a lower arm, an upper leg and a lower leg, truncal, pelvis, and/or on a helmet as described with reference to FIG. 1D). For example, the multiple DPAGA systems 101c may be used for detection of health issues, for example, including sport/injury detection, disease identification, evidence of fatigue, or evidence of lacking rest/sleep. For example, the DPAGA system 101c may advantageously be used to monitor health information for critical personnel (e.g., soldiers, pilots, truck drivers). In some examples, the DPAGA system 101c may be used to monitor medication side effects and/or effectiveness. For example, the DPAGA system 101c may be used to monitor a Parkinson disease drug for a Parkinson disease patient.

In some implementations, the DPAGA system 101c may be placed within a brace and/or casts to detect swelling. For example, the DPAGA system 101c may advantageously be used to detect an early infection during healing or a development of complications (e.g., nerve injuries, Complex Regional Pain Syndrome). In some implementations, the DPAGA system 101c may be small in size that may advantageously be placed in implants in a body. For example, a computer communicationally coupled to the 101c via the communication module 175 may be used to track shifting of spinal cord stimulator or recovery from knee and/or hip surgeries.

In some implementations, the DPAGA system 101c may be used to monitor sleeping pattern of the user 100. For example, the DPAGA system 101c may advantageously detect movement. For example, the DPAGA system 101c may be used to predict decubitus ulcer. For example, the DPAGA system 101c may be used to predict metabolic indication. For example, the DPAGA system 101c may be used to predict subcutaneous (e.g., by glucose detection).

FIG. 2 is an anterior view of a patient's lower right extremity. FIG. 3 is a posterior perspective view of a patient's lower right extremity. In some implementations, the wearable vibration device 104 may be applied at a position on an ankle of the user 100. In some examples, the actuators 150 may be positioned proximate to the patient's medial malleolus 108 or lateral malleolus 112 to effectively provide gait assistance to the user 100.

FIG. 4 is a plan view of an illustrative embodiment of an exemplary wearable vibration device shown on an exterior side. In this example, the wearable vibration device 104 includes an elongated strap 116 having a first end 120 and a second end 124. In some implementations, the strap 116 may be sown from industrial strength material. The strap 116, for example, may be configured to accommodate different size ankles by having a plurality of fasteners 128 on the first end 120, and the complementary plurality of fasteners 132 on the second end 124. The plurality of fasteners 128, 132 may include snaps, hook and loop fasteners, buckles and accommodating apertures, tying strings, and/or other fasteners. In this example, the strap 116 includes snaps 136 for fastening around an ankle in a closed position.

FIG. 5 is a plan view of the wearable vibration device of FIG. 4 shown on an interior side. As shown, one side of the strap 116 in the closed position is an exterior 140 and the other side is an interior 144. A circuit 148 may be attached to the strap 116 on the exterior 140 or in an interior cavity portion formed by layers that comprise the strap in some embodiments. Actuators 150 is coupled on the interior 144 or in the interior cavity portion. In some implementations, in the closed position of the wearable vibration device 104, the actuators 150 may be positioned proximate to the patient's medial malleolus 108 (as shown in FIG. 2) or lateral malleolus 112 (as shown in FIG. 2). The actuators 150 may be, for example, a micro-vibration motor such as a coin-battery vibration motor or other type of motor or vibration source to provide vibratory stimulus to the patient's bone proximate the ankle or lower leg, and in one embodiment to the medial or lateral malleolus. While only one actuator is shown, in this example, one or more actuators 150 or vibration engines 190a might also be used in some implementations.

In various implementations, the circuit 148 may include any number of components to provide power to the actuators 150 when desired. Some embodiments may only activate the actuators 150 when the user 100 is in the process of standing as indicated by an accelerometer or experiencing a gait impairment. For example, the controller 1005a may detect the patient in the process of standing based on sensor input and/or historical data based on the classification sub-model 1020. In this example, the circuit 148 includes a battery 152, such as a lithium-ion battery that is coupled to a control circuit board 156 or module, a communications module 160, such as a Bluetooth chip, and an accelerometer 164 (e.g., the force sensor 180b). For example, the accelerometer 164 may be disposed on an inside perimeter to detect positioning of the device as a person moves their limb.

In some implementations, the force sensor 180b (e.g., the accelerometer 164) may be inward facing (e.g., in contact with the ankle). For example, an inward facing accelerometer 164 may advantageously be used to determine positioning of the ankle of a user. For example, the inward facing accelerometer 164 may also advantageously be used to analyze tendon/soft tissue behavior (e.g., to detect changes that may predict improvement or pathology (e.g., edema, changes in soft tissue/ tendon behavior that precede a clinically apparent injury).

The communications module 160 may allow communication to the controller 1005a, and/or a computing device, such as a smartphone 170. The control circuit board 156 is electrically and/or communicatively coupled to the actuators 150, such as by conducting wires 168. In some implementations, the circuit 148 may include the accelerometer 164 for detecting movement (e.g., multiple actual actions) of the patient's leg. For example, the accelerometer 164 may be a 9-Degree of Freedom Inertial Measurement Unit (9 DOF IMU). In some implementations, the circuit 148 may include a 3-axis accelerometer, 3-axis magnetometer, 3-axis gyroscope. For example, the circuit 148 may include an onboard absolute orientation feature to detect yaw, pitch, and roll motions.

In one illustrative embodiment, the wearable vibration device 104 contains the accelerometer 164 that tracks movement of the user and allows the circuit 148 to activate when the patient is in the process of standing up and turns off when the patient is sitting down. In some embodiments, machine learning can be used to learn the patient's gait patterns and watch for issues such as potential falls or a heightened need for stability; the circuit 148 can determine that the patient is about to fall and apply different levels of intensity to the actuators 150 based on need.

In one illustrative embodiment, gait information from the accelerometer 164 and movement as tracked by the circuit 148 may be transmitted to the cloud system 1010 to track the user's gait over time. For example, the cloud system 1010 may track the user's gait over time to observe a deterioration pattern. In some examples, the cloud system 1010 may use the received data to train the machine learning model 1015. In some implementations, the rate of decline as well as possibility of falls can be monitored. In the instance of potential falls, in some implementations, the controller 1005a may use a feedback loop between the sensors 1035 and the actuators 150 to modulate the stimulation to lessen the probability of fall in a dynamic fashion.

In some implementations, the wearable vibration device 104 further includes one or more of the following: a heart rate sensor, temperature sensor, moisture level sensor, EKG, pulse oximeter and sweat composition sensor. For example, one or more of these additional sensors may generate training data to the cloud system 1010. Autonomic instability, for example, may be detected with heart rate variability (e.g., changes in pulse) and/or changes in limb temperature. The cloud system 1010 may train the machine learning model 1015 to, in some embodiments, use data received from various sensors of the wearable vibration device 104 to track fatigue or dehydration of an athlete or a soldier, for example.

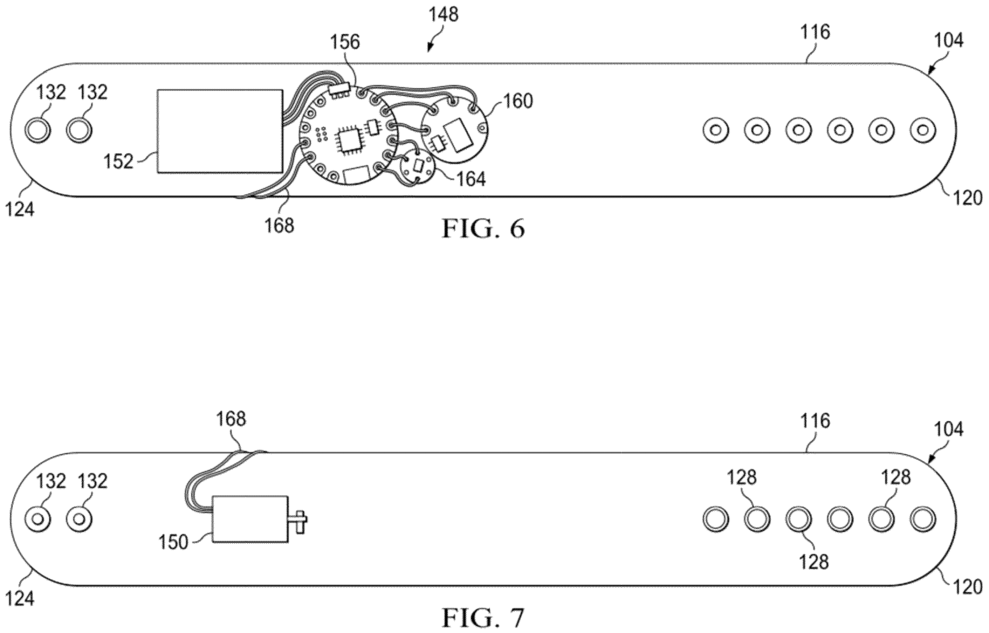

FIG. 6 is a schematic plan view on an exterior of an illustrative embodiment of a wearable vibration device 104. FIG. 7 is a schematic plan view on an interior side of the wearable vibration device 104 of FIG. 6. In various implementations, the wearable vibration device 104 may determine locally to selectively activate a vibration gait assistance mode.

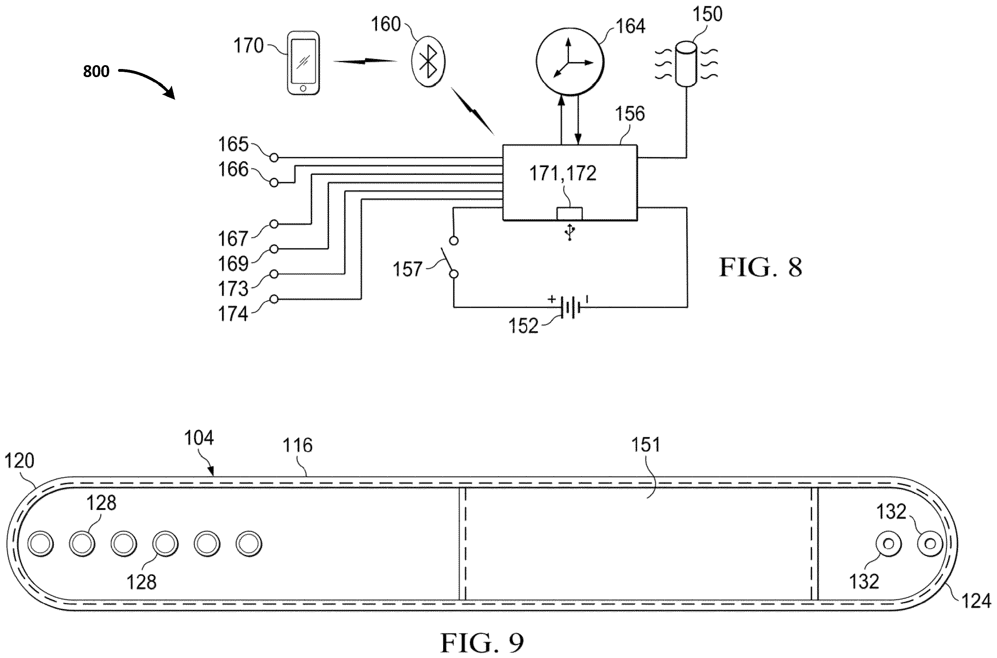

FIG. 8 is a schematic diagram of an illustrative embodiment of an exemplary circuit 800 for use as an aspect of an illustrative embodiment of a wearable vibration device 104. As shown, the control circuit board 156 includes a plurality of I\O ports 165-167,169,173,174. For example, the I\O ports may be connected to a plurality of sensors 165-167,169,173,174. The circuit further includes local comm ports 171,172 (e.g., a micro-USB port) for connection to a local computer, for example. The circuit 800 also includes the Bluetooth communication module 160 to transmit signals to the smartphone 170. In other embodiments, the circuit 148 may be formed as a flexible, stretchable circuit.

FIG. 9 is a plan view of an illustrative embodiment of a wearable vibration device 104 shown on an exterior side. For example, the strap 116 may include a medical grade silicone wristband. In some embodiments, the strap 116 may include a disposable strap releasably coupled to a control circuit (e.g., the exemplary circuit 800). For example, the strap 116 may attach to an electronics case of the exemplary circuit 800. Accordingly, for example, the exemplary circuit 800 may advantageously be removed between patients.

FIG. 10 is a plan view of an illustrative embodiment of a remote-controlled wearable vibration device 104 shown on an interior side. In this example, the remote-controlled wearable vibration device 104 includes an antenna 200 coupled to a controller 204 that may include a plurality of subsidiary circuits. The controller 204 may be electrically coupled to a battery 152, e.g., 3.7V battery, and to the actuators 150. The antenna may be used to receive an activation signal from a remote control 208 in FIG. 11. For example, the remote control 208 may include the user interface 185 described with reference to FIG. 1B. The remote control 208 may include a plurality of selectors 212.

FIG. 11 is a block diagram of an exemplary remote controlled wearable vibration device. As shown, the remote control 208 includes a plurality of selectors 212. In some implementations, the selectors 212 may provide user feedback to the controller 1005a. For example, when the wearable vibration device 104 is activated at an erroneous timing, (e.g., the user 100 does not need the vibration motor to activate), the user 100 may select one of the selectors 212 to provide feedback to the controller 1005a. In some examples, when the user 100 desires or feels that gait assistance is needed, the user 100 may select one of the selectors 212 to remotely activate the actuators 150. In some implementations, the controller 1005a and the cloud system 1010 may use the patient's feedback to further train their classification sub-model 1020 and machine learning model 1015 accordingly.

In some implementations, a DPAGA system (the DPAGA systems 101, 101b, 101c, ,102) may be included in a shoe. For example, the DPAGA system may include sensors (e.g., the one or more sensors 1035) for measuring parameters of an environment. The DPAGA may, for example, include the wearable vibration device 104. In some examples, the shoe may be used to replace a blind walking stick and/or a seeing eye dog.

In some implementations, the actuators 150 may include multiple haptic actuators. For example, four haptic actuators may include (e.g., at an anterior, a posterior, both lateral sides, or a combination thereof). Various embodiments may advantageously increase vibration intensities. For example, the vibration may be used as a directional cue.

FIG. 12 is a flow diagram showing an exemplary method 1200 for providing AI based gait assistance. For example, the method 1200 may be executed by the controller 1005a paired with the wearable vibration device 104. In this example, the method 1200 starts when data are received from sensors of a paired wearable vibration device in step 1205. Next, in step 1210, a user's need is classified for gait assistance. For example, the controller 1005a may use the classification sub-model 1020 to run a classification algorithm to classify whether the user 100 needs a gait assistance based on the received data.

In step 1215, it is determined whether the user needs gait assistance. If it is determined that the user does not need gait assistance, then the method 1200 continues monitoring in step 1220 and returns to the step 1205. If it is determined that the user needs gait assistance, then a signal is sent to activate a motor for gait assistance in step 1225 and the method 1200 ends. For example, the activation module 1025 may send a signal to the actuators 150 to provide gait assistance.

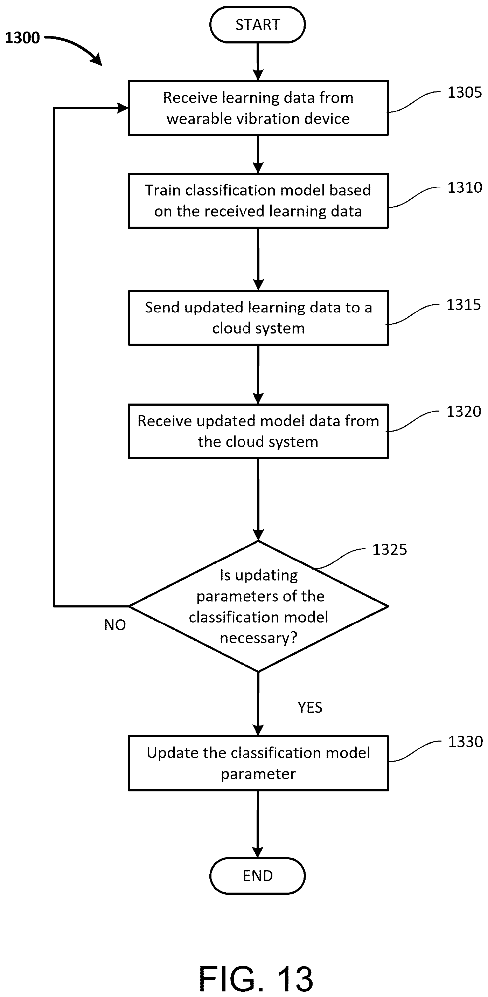

FIG. 13 is a flow diagram of an exemplary training method 1300 for providing ML based gait assistance. For example, the method 1200 may be executed by the controller 1005a paired with the wearable vibration device 104. The method 1300 starts when learning data is received from sensors of a paired wearable vibration device in step 1305. For example, the learning data may be user feedback. Next, in step 1310, a classification model is trained based on the received learning data. For example, the controller 1005a may use a user feedback signal to train the classification sub-model 1020. In step 1315, updated learning data is sent to a cloud system. For example, the controller 1005a may send the updated learning data to the cloud system 1010 to update the machine learning model 1015. Updated model data is, in step 1320, received from the cloud system.

After the updated model data is received, it is determined whether updating parameters of the classification model is necessary in step 1325. For example, the controller 1005a may compare a classification error to a predetermined threshold. If the classification error is greater than a predetermined threshold, then it is determined that updating parameters of the classification model is necessary. If it is determined that updating parameters of the classification model is not necessary, then the method 1300 returns to step 1305. If it is determined that updating parameters of the classification model is necessary, then the classification model parameter(s) are updated in step 1330. Then the method 1300 ends.

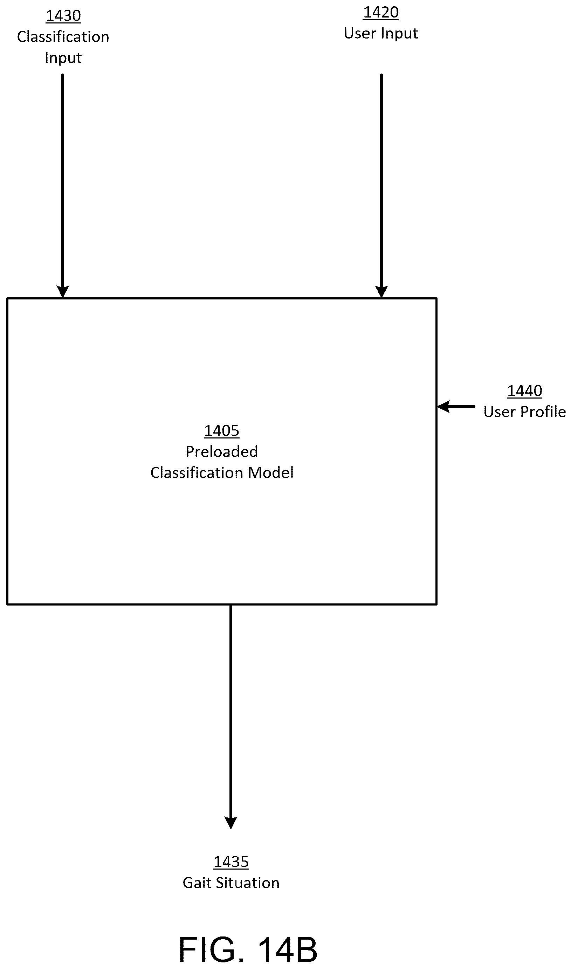

FIG. 14A and FIG. 14B are block diagrams of an exemplary gait assistance device 1400 including a preloaded classification model 1405. In some implementations, the DPAGA system 101 may perform operations described with reference to the gait assistance device 1400. For example, the preloaded classification model 1405 may be preloaded in a configuration process before selling to a user (e.g., the user 100).

As shown in FIG. 14A, the gait assistance device 1400 includes controller 1401. The controller 1005a includes the activation module 1025 and the preloaded classification model 1405. In some implementations, the activation module 1025 may apply classification input including sensor data from the one or more sensors 1035 and a user input 1420 (optional) to the preloaded classification model 1405 to generate a gait situation. Based on the gait situation, the activation module 1025 may selectively activate the actuators 150.

In some implementations, the exemplary gait assistance device 1400 may optionally be connected to a gait assistance training system 1410. For example, the gait assistance training system 1410 may provide software updates, including update to the preloaded classification model 1405. For example, the exemplary gait assistance device 1400 may include a communication module 175 configured to connect to the gait assistance training system 1410 over a network. For example, the exemplary gait assistance device 1400 may include a data communication port (e.g., a USB port) to receive data from the gait assistance training system 1410 (e.g., through a portable media).

In some implementations, the gait assistance training system 1410 may include a machine learning model 1425. For example, the gait assistance training system 1410 may receive centralized training data 1415 to train the user input 1420. For example, the centralized training data 1415 may be a common data set across multiple exemplary gait assistance devices 1400. Accordingly, the exemplary gait assistance device 1400 may advantageously use a uniformly trained and verified preloaded classification model 1405 for gait assistance.

As shown in FIG. 14B, a block diagram of an exemplary preloaded classification model 1405 is shown. In this example, the preloaded classification model 1405 receives a classification input 1430 and the user input 1420. For example, the 1430 may include sensor data from the ambient sensor 180a, the force sensor 180b, and the bio-sensor 180c. As a function of the user input 1420 and the classification input 1430, the preloaded classification model 1405 generates a gait situation 1435. For example, the 1435 may include that the user 100 does not need gait assistance, needs stronger gait assistance, or needs less vigorous gait assistance.

In some implementations, as shown in FIG. 14B, the preloaded classification model 1405 may also determine the gait situation 1435 based on a user profile 1440. For example, the user profile 1440 may include a demographic of a user (e.g., age, health conditions, gender). In some examples, the user profile 1440 may include historical user behaviors. For example, some of the historical user behaviors may be associated with a current time. For example, the preloaded classification model 1405 may classify at 10pm that a user is typically asleep, and generate a gait situation that activates illumination guidance during 10pm - 5am.

FIG. 15 is a flow diagram of an exemplary pre-training method 1500 for providing AI based gait assistance by an exemplary gait assistance device. A method 1500 may, for example, be performed by a processor(s) (e.g., in the gait assistance training system 1410) executing a program(s) of instructions retrieved from a data store(s) (e.g., data store 145). The method 1500 includes, at a step 1515, receiving training data (e.g., the centralized training data 1415). For example, the training data may include an input vector of classification input (e.g., force sensor measurements, ambient sensor measurements, bio-sensor measurements) and a corresponding gait situation output.

At a step 1520, the retrieved data is divided into a first set of data used for training and a second set of data used for testing. At a step 1525, a model (e.g., an initial model of the user input 1420) is applied to the training data to generate a trained model (e.g., a neural network model, a random forest, a supervised classification model). The trained model is applied to the testing data, in a step 1530, to generate test output(s) (e.g., content attribute profile(s)). The output is evaluated, in a decision point 1535, to determine whether the model is successfully trained (e.g., by comparison to a predetermined training criterion(s)). The predetermined training criterion(s) may, for example, be a maximum error threshold. For example, if a difference between the actual output (the test data) and the predicted output (the test output) is within a predetermined range, then the model may be regarded as successfully trained. If the difference is not within the predetermined range, then the model may be regarded as not successfully trained. At a step 1540, the processor may generate a signal(s) requesting additional training data, and the method 1500 loops back to step 1530. If the model is determined, at the decision point 1535, to be successfully trained, then the trained model may be stored (e.g., in the storage module 225), in a step 1545, and the method 700 ends.

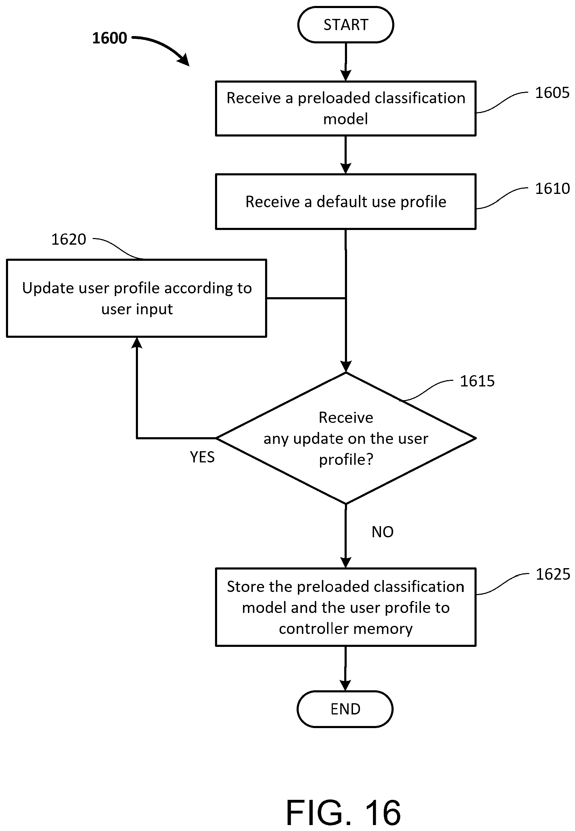

FIG. 16 is a flow diagram of an exemplary configuration method 1600 for providing AI based gait assistance by an exemplary gait assistance device. For example, the method 1600 may be performed by the controller 1401 when the exemplary gait assistance device 1400 is manufactured. In this example, the method 1600 begins when a preloaded classification model is received in step 1605. For example, the controller 1401 may receive the preloaded classification model 1405 from the gait assistance training system 1410.

Next, in step 1610, a default user profile is received. For example, the default user profile may include a default age, gender, and/or health conditions of a user. In a decision point 1615, it is determined whether any update is received for the user profile. For example, the exemplary gait assistance device 1400 may be sold to a known person such that the actual conditions of the person may be input into the exemplary gait assistance device 1400. If there are some updates to the user profile, in step 1620, the user profile is updated according to the user input, and the decision point 1615 is repeated. If there is no update to the user profile, in step 1625, the preloaded classification model and the user profile are stored to a controller memory (e.g., in the controller 1401), and the method 1600 ends.

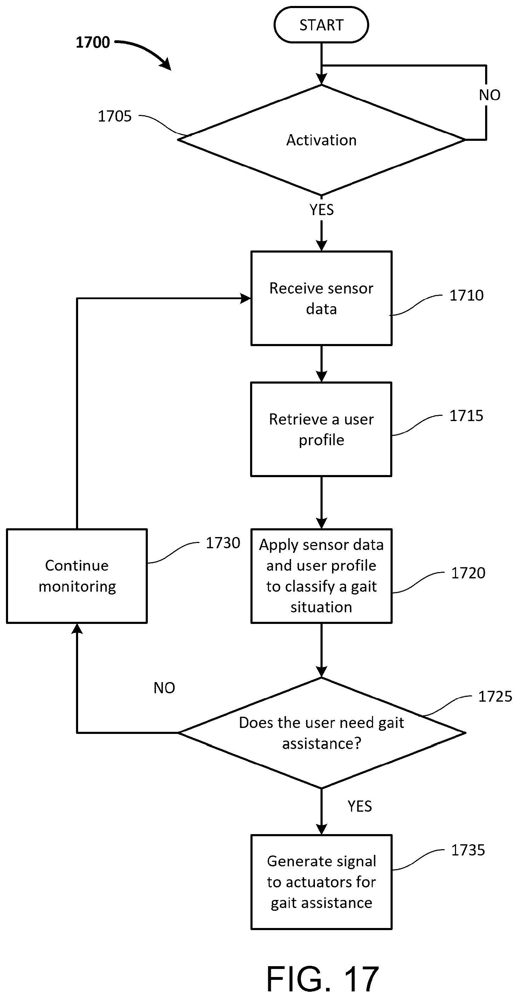

FIG. 17 is a flow diagram of an exemplary assistance method 1700 for gait impaired users using an exemplary gait assistance device. For example, the controller 1401 may perform the method 1700 to assist a gait impaired user. In some implementations, the method 1700 may also be performed by the controller 1005a in an offline mode. In this example, the method 1700 begins by determining, in a decision point 1705, whether a gait assistant device is activated. If the gait assistant device is not activated, the decision point 1705 is repeated.

If the gait assistant device is activated, in step 1710, sensor data is received. For example, the sensor data may be received from the one or more sensors 1035. Next, a user profile is retrieved in step 1715. For example, the user profile may be retrieved from the controller memory as described with reference to FIG. 16. In step 1720, the sensor data and the user profile ares applied to classify a gait situation. For example, the classification input 1430 and the user profile 1440 may be applied to the preloaded classification model 1405 to generate the gait situation 1435.

In a decision point 1725, it is determined whether the user needs gait assistance. If it is determined that the user does not need gait assistance, then the method 1700 continues monitoring in step 1730 and returns to the step 1710. If it is determined that the user needs gait assistance, then a signal is sent to activate actuators for gait assistance in step 1735 and the method 1700 ends. For example, the activation module 1025 may send a signal to the actuators 150 to provide vibration gait assistance, vision guidance, audio guidance, or a combination thereof.

Although various embodiments have been described with reference to the figures, other embodiments are possible. In some implementations, the circuit 148 may include a timing circuit that vibrates on a periodic basis to call the wearer to attention or for other purposes. For example, either a time vibration signal or controlled vibration signal may be periodically delivered that will assist the user in regaining focus. For example, attention deficit disorders may potentially be positively impacted by sensory stimulation which can help children and/or adults focus.

In some embodiments, the user 100 may have a wearable vibration device 104 on each leg. For example, multiple devices may be used for dystonia, tremors, and/or pain.

In some implementations, the DPAGA system 101 may include additional or different input points. For example, in one embodiment, the DPAGA system 101 may include additional devices with multiple vibration motors to stimulate vibration conduction through various nerves throughout the hand. In various applications, the following illustrative contact points may, by way of example and not limitation, be used to apply vibration with a wearable vibration device 104: Skull (occipital protuberance, temple, angle of jaw, zygomatic arch, forehead, mastoid); Spine (Vertebral processes (in the cervical/thoracic/lumbar spine); thorax (Clavicle, Costoclavicular processes, Sternum, Ribs); Shoulders (Acromion, spine of the scapula); Arms (Elbow: Medial/lateral epicondyle (epicondylitis), olecranon, a touch point over Guyon's canal for ulnar neuropathy); Wrist/hand (lister's tubercle as well as the bones of the wrist/hand, focus over the components of the carpal tunnel, a glove for osteoarthritis where the vibration tool lines up with the joints of the digits); Pelvis (iliac crest, anterior superior iliac spine, anterior inferior iliac spine, pubic bone, inferior pubic ramus, Sacrum, coccyx); Legs (Greater trochanter of hip, Knee - touch point at femur (anteriorly, laterally and posteriorly), tibia (anteriorly, laterally and posteriorly), patella); Ankle - at the lateral malleoli and anteriorly; Heel; Foot - multiple locations including first metatarsophalangeal joint (gout); genitalia, and/or orifices (oral, rectal, introital); or some combination thereof.

In some embodiments, for joint replacements and/or other implantables such as spinal fusions or total knee replacements, the DPAGA system 101 may include a vibratory component in the implant and that can be used as a pain management tool. In the knee implant (total knee replacement) or hip replacement (total hip replacement), a vibration tool and accelerometer may, for example, be applied so that pain relief is provided, and function can be tracked (e.g., restoring/maintaining range of motion may be key to success with such surgeries).

In another alternative embodiment, a vibration device can be included in fasteners used in spinal surgery. The device can be in the screws that are drilled into the spine; the vibratory component can provide pain management. Also having an accelerometer may help to detect range of motion including whether there is pathological movement (early indicator of non-union).

In another embodiment, for other surgeries across joints (shoulder, elbow, wrist, knee, ankle), an external sleeve with a vibratory motor could be used; the accelerometer and bone vibration could detect movement and provide pain relief. Some combination of a heart rate sensor, temperature sensor, moisture level sensor, pulse oximeter and sweat composition sensor could monitor for complications of surgery.

In another embodiment, the vibratory motor and accelerometer may, for example, be placed in a cast to provide pain relief for the restricted body part and detect pathological healing. In some implementations, one of more of the following sensors may, by way of example and not limitation, be used to monitor for abnormal healing/complications of fractures (e.g., including complex regional pain syndrome): a heart rate sensor, temperature sensor, moisture level sensor, pulse oximeter, sweat composition sensor, or some combination thereof.

In an alternative embodiment, a vibration device similar in function to the wearable vibration device 104 could be an implantable that could be placed adjacent to the bony structures described above to provide relief of gait impairment, movement disorders and pain as described above.

The devices, systems and methods presented here for gait impairment or certain forms of pain control may, for example, advantageously not require surgery for application and/or may, for example, advantageously be quickly applied to a patient. Moreover, such devices may, for example, be comfortable to wear.

For example, the wearable vibration device 104 may be used for application to a patient. For example, the wearable vibration device may include a strap that is sized and configured to fit on the patient's ankle. For example, the actuators 150 may be coupled to the strap such that when the strap is in an applied position on the patient's ankle. In some examples, the actuators 150 may be positioned proximate to the patient's medial malleolus or lateral malleolus. In some implementations, the circuit for powering the actuators 150 may include an accelerometer for detecting movement of the patient's leg, and a battery for supplying energy to the circuit and vibration motor. In some examples, the wearable vibration device 104 may also include a communications module for providing wireless signals to a smartphone or monitoring computer.

In some implementations, the DPAGA system 101 may provide a method of treating a patient with gait impairment by providing a wearable vibration device to the patient's ankle. For example, the method may activate a vibration motor on the wearable vibration device to help provide proprioception for the patient.

In some implementations, the DPAGA system 101 may provide a method for treating pain, the method comprising applying a wearable vibration device to an extremity of a patient to supply painless input in the form of vibration to utilize gait control theory to reduce pain.

In some implementations, the DPAGA system 101 may provide a method of treating a patient with a movement disorder. For example, the method may include placing a wearable vibration device on one or more extremities of the patient. For example, the wearable vibration device 104 may be selectively activated to accomplish a sensory 'trick' (e.g., providing artificial stimulus augmenting or replacing stimulus that would normally be naturally provided during walking).

In some implementations, the wearable vibration device 104 may, for example, include a housing that is sized and configured for implantation and placement proximate to a bone of the patient, a vibration motor coupled to the housing, the actuators 150 may be positioned proximate the patient's bone; and a circuit for powering the vibration motor. For example, the circuit may include an accelerometer for detecting movement of the patient, and a battery for supplying energy to the circuit and vibration motor.

The wearable vibration device 104 may be applied at various and/or multiple locations for a therapeutic outcome. In one embodiment, the wearable vibration device 104 may be applied to the body at the ankle with a Bluetooth remote on/off so it can be operated remotely. The addition of an accelerometer with tracking of movement may, for example, allow the tracking of movement which could provide insight into relaxation vs. restlessness/ agitation.

Although an exemplary system has been described with reference to FIGS. 1A-17, other implementations may be deployed in other industrial, scientific, medical, commercial, and/or residential applications.

The wearable vibration device 104 may, for example, be applicable to one or more indications or uses. As an illustrative example, the wearable vibration device may, for example, be used with patients having movement disorders (e.g., Cervical dystonia, Parkinson's). With movement disorders, people may have uncontrollable movement and/or may have a difficult time initiating an activity (such as walking). Accordingly, a "sensory trick" may, for example, be used. The sensory trick may, for example, be visual and/or physical. In such situations, the patient feels something or sees something, and it helps them arrest an uncontrolled movement or initiate motion such as getting up, turning over in bed or walking. In one embodiment, a patient may, for example, wear the wearable vibration device 104 that (e.g., with the accelerometer) may detect when the patient has stopped, and the vibration may then be applied as a sensory trick to encourage movement. In this application, the wearable vibration device 104 may be applied to other extremities (e.g., such as the wrists) in certain situations and/or on multiple extremities.

The wearable vibration device 104 may also be used as a sensory trick to help with action tremors. In that situation, the wearable vibration device 104 may, for example, be applied on both sides. The wearable vibration device 104 may, for example, be applied on the wrists.

In another embodiment, a wearable vibration device 104 may, by way of example and not limitation, be placed over the cervical spine and/or clavicle (e.g., to arrest symptoms of dystonia).

In one embodiment, the wearable vibration device 104 may include one or more sensors of the type(s) previously mentioned. For tremors, for example, the wearable vibration device 104 may be used to track a response to medications and/or other treatments (such as implantable).

For example, the wearable vibration device 104 may be used by a patient having pain. Without being limited to a particular theory, under the gate control theory of pain, a non-painful input, e.g., vibration in this embodiment, may close the nerve "gates" to painful inputs, which may, for example, prevent pain sensation from traveling to the central nervous system. For example, by placing the wearable vibration device 104 on the ankles and allowing the vibration to be a source of non-painful input, the wearable vibration device 104 may be configured to help relieve pain.

Another indication for a wearable vibration device 104 may, for example, include treatment of a number of sensory perception /behavioral/psychological disorders. For example, the wearable vibration device 104 may be configured to provide sensory stimulation for sensory processing disorders. In some examples, the wearable vibration device 104 may, for example, be configured for application to other disorders that may respond to sensory stimulation (e.g., anxiety, depression, post-traumatic stress disorder (PTSD)).

The wearable vibration device 104 may, for example, be applied at various or multiple locations for a therapeutic outcome. In one embodiment, a wearable device may be applied, for example, to the body at the ankle with a Bluetooth remote on/off so it can be operated remotely. The addition of an accelerometer with tracking of movement may, for example, allow the tracking of movement which could provide insight into relaxation vs. restlessness/agitation

In various embodiments, some bypass circuits implementations may be controlled in response to signals from analog or digital components, which may be discrete, integrated, or a combination of each. Some embodiments may include programmed, programmable devices, or some combination thereof (e.g., PLAs, PLDs, ASICs, microcontroller, microprocessor), and may include one or more data stores (e.g., cell, register, block, page) that provide single or multi-level digital data storage capability, and which may be volatile, non-volatile, or some combination thereof. Some control functions may be implemented in hardware, software, firmware, or a combination of any of them.

Computer program products may contain a set of instructions that, when executed by a processor device, cause the processor to perform prescribed functions. These functions may be performed in conjunction with controlled devices in operable communication with the processor. Computer program products, which may include software, may be stored in a data store tangibly embedded on a storage medium, such as an electronic, magnetic, or rotating storage device, and may be fixed or removable (e.g., hard disk, floppy disk, thumb drive, CD, DVD).

Although an example of a system, which may be portable, has been described with reference to the above figures, other implementations may be deployed in other processing applications, such as desktop and networked environments.

Temporary auxiliary energy inputs may be received, for example, from chargeable or single use batteries, which may enable use in portable or remote applications. Some embodiments may operate with other DC voltage sources, such as a 9V (nominal) batteries, for example. Alternating current (AC) inputs, which may be provided, for example from a 50/60 Hz power port, or from a portable electric generator, may be received via a rectifier and appropriate scaling. Provision for AC (e.g., sine wave, square wave, triangular wave) inputs may include a line frequency transformer to provide voltage step-up, voltage step-down, and/or isolation.

Although particular features of an architecture have been described, other features may be incorporated to improve performance. For example, caching (e.g., L1, L2, ...) techniques may be used. Random access memory may be included, for example, to provide scratch pad memory and or to load executable code or parameter information stored for use during runtime operations. Other hardware and software may be provided to perform operations, such as network or other communications using one or more protocols, wireless (e.g., infrared) communications, stored operational energy and power supplies (e.g., batteries), switching and/or linear power supply circuits, software maintenance (e.g., self-test, upgrades), and the like. One or more communication interfaces may be provided in support of data storage and related operations.

Some systems may be implemented as a computer system that can be used with various implementations. For example, various implementations may include digital circuitry, analog circuitry, computer hardware, firmware, software, or combinations thereof. Apparatus can be implemented in a computer program product tangibly embodied in an information carrier, e.g., in a machine-readable storage device, for execution by a programmable processor; and methods can be performed by a programmable processor executing a program of instructions to perform functions of various embodiments by operating on input data and generating an output. Various embodiments can be implemented advantageously in one or more computer programs that are executable on a programmable system including at least one programmable processor coupled to receive data and instructions from, and to transmit data and instructions to, a data storage system, at least one input device, and/or at least one output device. A computer program is a set of instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

Suitable processors for the execution of a program of instructions include, by way of example, both general and special purpose microprocessors, which may include a single processor or one of multiple processors of any kind of computer. Generally, a processor will receive instructions and data from a read-only memory or a random-access memory or both. The essential elements of a computer are a processor for executing instructions and one or more memories for storing instructions and data. Generally, a computer will also include, or be operatively coupled to communicate with, one or more mass storage devices for storing data files; such devices include magnetic disks, such as internal hard disks and removable disks; magneto-optical disks; and optical disks. Storage devices suitable for tangibly embodying computer program instructions and data include all forms of non-volatile memory, including, by way of example, semiconductor memory devices, such as EPROM, EEPROM, and flash memory devices; magnetic disks, such as internal hard disks and removable disks; magneto-optical disks; and CD-ROM and DVD-ROM disks. The processor and the memory can be supplemented by, or incorporated in, ASICs (application-specific integrated circuits).

In some implementations, each system may be programmed with the same or similar information and/or initialized with substantially identical information stored in volatile and/or non-volatile memory. For example, one data interface may be configured to perform auto configuration, auto download, and/or auto update functions when coupled to an appropriate host device, such as a desktop computer or a server.

In some implementations, one or more user-interface features may be custom configured to perform specific functions. Various embodiments may be implemented in a computer system that includes a graphical user interface and/or an Internet browser. To provide for interaction with a user, some implementations may be implemented on a computer having a display device, such as a CRT (cathode ray tube) or LCD (liquid crystal display) monitor for displaying information to the user, a keyboard, and a pointing device, such as a mouse or a trackball by which the user can provide input to the computer.

In various implementations, the system may communicate using suitable communication methods, equipment, and techniques. For example, the system may communicate with compatible devices (e.g., devices capable of transferring data to and/or from the system) using point-to-point communication in which a message is transported directly from the source to the receiver over a dedicated physical link (e.g., fiber optic link, point-to-point wiring, daisy-chain). The components of the system may exchange information by any form or medium of analog or digital data communication, including packet-based messages on a communication network. Examples of communication networks include, e.g., a LAN (local area network), a WAN (wide area network), MAN (metropolitan area network), wireless and/or optical networks, the computers and networks forming the Internet, or some combination thereof. Other implementations may transport messages by broadcasting to all or substantially all devices that are coupled together by a communication network, for example, by using omni-directional radio frequency (RF) signals. Still other implementations may transport messages characterized by high directivity, such as RF signals transmitted using directional (i.e., narrow beam) antennas or infrared signals that may optionally be used with focusing optics. Still other implementations are possible using appropriate interfaces and protocols such as, by way of example and not intended to be limiting, USB 2.0, Firewire, ATA/IDE, RS-232, RS-422, RS-485, 802.11 a/b/g, Wi-Fi, Ethernet, IrDA, FDDI (fiber distributed data interface), token-ring networks, multiplexing techniques based on frequency, time, or code division, or some combination thereof. Some implementations may optionally incorporate features such as error checking and correction (ECC) for data integrity, or security measures, such as encryption (e.g., WEP) and password protection.

In various embodiments, the computer system may include Internet of Things (IoT) devices. IoT devices may include objects embedded with electronics, software, sensors, actuators, and network connectivity which enable these objects to collect and exchange data. IoT devices may be in-use with wired or wireless devices by sending data through an interface to another device. IoT devices may collect useful data and then autonomously flow the data between other devices.

Various examples of modules may be implemented using circuitry, including various electronic hardware. By way of example and not limitation, the hardware may include transistors, resistors, capacitors, switches, integrated circuits, other modules, or some combination thereof. In various examples, the modules may include analog logic, digital logic, discrete components, traces and/or memory circuits fabricated on a silicon substrate including various integrated circuits (e.g., FPGAs, ASICs), or some combination thereof. In some embodiments, the module(s) may involve execution of preprogrammed instructions, software executed by a processor, or some combination thereof. For example, various modules may involve both hardware and software.

In an illustrative aspect, a gait assisting apparatus may include a coupling module configured to couple to a user, and a wearable vibration device. The wearable vibration device may include a housing coupled to the coupling module, a sensor module configured to generate a sensor measurement from measured data associated with the user, and an actuator configured to provide gait assistance to the user. For example, the actuator may include a vibration module configured to generate vibration gait assistance, and a vision assistance module configured to generate an illumination guidance.

The wearable vibration device may include a controller operably coupled to the sensor module, may include an activation module and a local learning model. For example, the local learning model may include a plurality of weighting configured to classify a gait situation based on a classification input, and the activation module may be configured to generate an activation level to control the actuator. For example, the wearable vibration device may include a communication module operably coupled to the controller, the communication module may be configured to connect to a remote federated machine learning model. For example, the remote federated machine learning model may be connected in data communication with a network of remote gait assisting apparatus.

For example, in an online mode, the local learning model may be configured to receive update weighting input from the remote federated machine learning model as a function of training inputs. For example, the training inputs may include updated training output parameters generated by the local learning models from the network of remote gait assisting apparatus based on sensor measurements from corresponding gait assisting apparatus.

For example, in an offline mode, upon receiving a sensor measurement from the sensor module, the activation module independently may apply the local learning model to the classification input may include the received sensor measurement to determine the activation level of the actuator such that the gait assisting apparatus may provide a gait assistant function to prevent gait impairment injuries in real time.

For example, the coupling module may include a medical grade silicone wristband. For example, the coupling module may include a disposable strap that may attach to the housing of the wearable vibration device. For example, the coupling module may be configured to a lower extremity of the user. For example, the housing may be releasably coupled to the coupling module.

For example, the sensor module may include a force sensor on an inside perimeter of the housing. For example, the force sensor may be configured to detect a relative position of the wearable vibration device to the user during limb movements. For example, the actuator may include an audio module configured to generate sound guidance to the user. For example, the activation level may include a plurality of intensity levels of gait assistance. For example, the classification input may include a current time and user input.

In an illustrative aspect, a stability assisting apparatus may include a coupling module configured to couple to a user, and a wearable vibration device. The wearable vibration device may include, for example, a housing coupled to the coupling module, a sensor module configured to generate a sensor measurement from measured data associated with the user, and an actuator configured to provide stability assistance to the user. For example, the actuator may include a vibration module configured to generate vibration stability assistance, and a vision assistance module configured to generate an illumination guidance. The wearable vibration device may include, for example, a controller operably coupled to the sensor module, may include an activation module and a local classification model. For example, the local classification model may include a plurality of weightings configured to classify a gait situation based on a classification input, and the activation module may be configured to generate an activation level to control the actuator.

For example, in operation, the activation module may apply the local classification model to the classification input may include received sensor measurements to determine the activation level of the actuator such that the stability assisting apparatus may provide a stability assistant function to prevent stability-related injuries in real time.

For example, the stability assistance to the user may include gait assistance. For example, the stability assistant function may include gait assistant function. For example, stability-related injuries may include gait injuries.

For example, the local classification model may include a machine learning model preloaded into the controller. For example, the machine learning model may be pre-trained with a common data set. For example, the stability assisting apparatus may include a communication module operably coupled to the controller. For example, the communication module may be configured to, upon receiving new weightings of the local classification model, update the local classification model.

For example, the coupling module may include a band configured to a limb of the user. For example, the coupling module may include a helmet. For example, the housing may be releasably coupled to the coupling module. For example, the sensor module may include a force sensor on an inside perimeter of the housing. For example, the force sensor may be configured to detect a relative position of the wearable vibration device to the user during limb movements.

In an illustrative aspect, a gait assisting apparatus may include a coupling module configured to couple to a user and a wearable vibration device. For example, the wearable vibration device may include a housing coupled to the coupling module, a sensor module configured to generate a sensor measurement from measured data associated with the user, an actuator may include a vibration module configured to generate vibration gait assistance to provide gait assistance, and a controller operably coupled to the sensor module. The controller, for example, may include an activation module and a local learning model.

For example, the local learning model may include a plurality of weightings configured to classify a gait situation based on a classification input, and the activation module may be configured to generate an activation level to control the actuator. For example, a communication module may operably be coupled to the controller. For example, the communication module may be configured to connect to a remote federated machine learning model. For example, the remote federated machine learning model may be connected in data communication with a network of remote gait assisting apparatus.

For example, in an online mode, the local learning model may be configured to receive update weighting input from the remote federated machine learning model as a function of training inputs. For example, the training inputs may include updated training output parameters generated by the local learning models from the network of remote gait assisting apparatus based on sensor measurements from corresponding gait assisting apparatus.

For example, in an offline mode, upon receiving a sensor measurement from the sensor module, the activation module independently may apply the local learning model to the classification input may include the received sensor measurement to determine the activation level of the actuator such that the gait assisting apparatus may provide a gait assistant function to prevent gait impairment injuries in real time.

For example, the actuator may include a vision assistance module configured to generate an illumination guidance. For example, the coupling module may include a disposable strap that may attach to the housing of the wearable vibration device. For example, the housing may be releasably coupled to the coupling module. For example, the sensor module may include a force sensor on an inside perimeter of the housing. For example, the force sensor may be configured to detect a relative position of the wearable vibration device to the user during limb movements.

A number of implementations have been described. Nevertheless, it will be understood that various modifications may be made. For example, advantageous results may be achieved if the steps of the disclosed techniques were performed in a different sequence, or if components of the disclosed systems were combined in a different manner, or if the components were supplemented with other components. Accordingly, other implementations are contemplated within the scope of the following claims.

## Claims

1. A gait assisting apparatus comprising:
a coupling module (116) configured to couple to a user; and,
a wearable vibration device (104) comprising:
a housing (195) coupled to the coupling module;
a sensor module (1035) configured to generate a sensor measurement from measured data associated with the user;
an actuator (150) configured to provide gait assistance to the user,
wherein the actuator comprises a vibration module (190a) configured to generate vibration gait assistance, and a vision assistance module (190b) configured to generate an illumination guidance;
a controller (1005a) operably coupled to the sensor module, comprising an activation module and a local learning model (1020), wherein the local learning model comprises a plurality of weighting configured to classify a gait situation based on a classification input, wherein a gait situation classification comprises no gait assistance and at least one intensity level of gait assistance, and the activation module (1025) is configured to generate an activation level to control the actuator based on the gait situation classification; and,
a communication module (175) operably coupled to the controller, the communication module is configured to connect to a remote federated machine learning model (1015), wherein the remote federated machine learning model is connected in data communication with a network of remote gait assisting apparatus (1005), such that,
in an online mode, the local learning model is configured to receive update weighting input from the remote federated machine learning model as a function of training inputs, wherein the training inputs comprise updated training output parameters generated by the local learning models from the network of remote gait assisting apparatus based on sensor measurements from corresponding gait assisting apparatus; and,
in an offline mode, upon receiving the sensor measurement from the sensor module, the activation module independently applies the local learning model to the classification input comprising the received sensor measurement to determine the activation level of the actuator such that the gait assisting apparatus provides a gait assistant function to prevent gait impairment injuries in real time.

2. The gait assisting apparatus of claim 1, wherein the coupling module comprises a medical grade silicone wristband.

3. The gait assisting apparatus of claim 1, wherein the coupling module comprises a disposable strap that attaches to the housing of the wearable vibration device.

4. The gait assisting apparatus of claim 1, wherein the coupling module is configured to a lower extremity of the user.

5. The gait assisting apparatus of claim 1, wherein the housing is releasably coupled to the coupling module.

6. The gait assisting apparatus of claim 1, wherein the sensor module comprises a force sensor on an inside perimeter of the housing, wherein the force sensor is configured to detect a relative position of the wearable vibration device to the user during limb movements.

7. The gait assisting apparatus of claim 1, wherein the actuator further comprises an audio module configured to generate sound guidance to the user.

8. The gait assisting apparatus of claim 1, wherein the activation level comprises a plurality of intensity levels of gait assistance.

9. The gait assisting apparatus of claim 1, wherein the classification input further comprises a current time and user input.

10. A stability assisting apparatus comprising:
a coupling module (116) configured to couple to a user; and,
a wearable vibration device (104) comprising:
a housing (195) coupled to the coupling module;
a sensor module (1035) configured to generate a sensor measurement from measured data associated with the user;
an actuator (150) configured to provide stability assistance to the user, wherein the actuator comprises a vibration module (190a) configured to generate vibration stability assistance, and a vision assistance module (190b) configured to generate an illumination guidance; and,
a controller (1401) operably coupled to the sensor module, comprising an activation module (1025) and a local classification model (1405), wherein the local classification model comprises a plurality of weightings configured to classify a gait situation based on a classification input (1430), wherein the gait situation comprises no gait assistance and at least one intensity level of gait assistance, and the activation module is configured to generate an activation level to control the actuator based on the gait situation classification, wherein:
in operation, the activation module applies the local classification model to the classification input comprising received sensor measurements to determine the activation level of the actuator such that the stability assisting apparatus provides a stability assistant function to prevent stability-related injuries in real time.

11. The stability assisting apparatus of claim 10, wherein the stability assistance to the user comprises a gait assistance, the stability assistant function comprises a gait assistant function, and the stability-related injuries comprises gait injuries.

12. The stability assisting apparatus of claim 10, wherein the local classification model comprises a machine learning model preloaded into the controller, wherein the machine learning model is pre-trained with a common data set.

13. The stability assisting apparatus of claim 10, further comprising a communication module operably coupled to the controller, wherein the communication module is configured to, upon receiving new weightings of the local classification model, update the local classification model.

14. The stability assisting apparatus of claim 10, wherein the coupling module comprises a band configured to a limb of the user.

15. The stability assisting apparatus of claim 10, wherein the coupling module comprises a helmet.

16. The stability assisting apparatus of claim 10, wherein the housing is releasably coupled to the coupling module.

17. The stability assisting apparatus of claim 10, wherein the sensor module comprises a force sensor on an inside perimeter of the housing, wherein the force sensor is configured to detect a relative position of the wearable vibration device to the user during limb movements.

18. A gait assisting apparatus comprising:
a coupling module (116) configured to couple to a user; and,
a wearable vibration device (104) comprising:
a housing (195) coupled to the coupling module;
a sensor module (1035) configured to generate a sensor measurement from measured data associated with the user;
an actuator (150) comprising a vibration module (190a) configured to generate vibration gait assistance to provide gait assistance;
a controller (1005a) operably coupled to the sensor module, comprising an activation module (1020) and a local learning model (1025), wherein the local learning model comprises a plurality of weightings configured to classify a gait situation based on a classification input, wherein the gait situation comprises no gait assistance and at least one intensity level of gait assistance, and the activation module is configured to generate an activation level to control the actuator based on the gait classification, wherein:
a communication module (175) operably coupled to the controller, the communication module is configured to connect to a remote federated machine learning model (1015), wherein the remote federated machine learning model is connected in data communication with a network of remote gait assisting apparatus (1005), such that,
in an online mode, the local learning model is configured to receive update weighting input from the remote federated machine learning model as a function of training inputs, wherein the training inputs comprise updated training output parameters generated by the local learning models from the network of remote gait assisting apparatus based on sensor measurements from corresponding gait assisting apparatus; and,
in an offline mode, upon receiving the sensor measurement from the sensor module, the activation module independently applies the local learning model to the classification input comprising the received sensor measurement to determine the activation level of the actuator such that the gait assisting apparatus provides a gait assistant function to prevent gait impairment injuries in real time.

19. The gait assisting apparatus of claim 18, wherein the actuator further comprises a vision assistance module configured to generate an illumination guidance.

20. The gait assisting apparatus of claim 18, wherein the coupling module comprises a disposable strap that attaches to the housing of the wearable vibration device.

21. The gait assisting apparatus of claim 18, wherein the housing is releasably coupled to the coupling module.

22. The gait assisting apparatus of claim 18, wherein the sensor module comprises a force sensor on an inside perimeter of the housing, wherein the force sensor is configured to detect a relative position of the wearable vibration device to the user during limb movements.

23. A stability assisting apparatus comprising:
a helmet (103) configured to couple to a user; and,
a wearable vibration device (104) comprising:
a housing (195) coupled to the helmet;
a sensor module (1035) coupled to the helmet and configured to generate a sensor measurement from measured data associated with the user;
an actuator (150) coupled to the helmet and configured to provide stability assistance to the user;
a controller (1401) coupled to the helmet and operably coupled to the sensor module, comprising an activation module (1025) and a local classification model (1405), wherein the local classification model comprises a plurality of weightings configured to classify a gait situation based on a classification input (1430), wherein a gait situation classification comprises no gait assistance and at least one intensity level of gait assistance, and the activation module is configured to generate an activation level to control the actuator based on the gait situation classification such that in-time assistance is selectively provided to the user when predicted to have trouble with gait, wherein:
in operation, the activation module applies the local classification model to the classification input comprising received sensor measurements to determine the activation level of the actuator such that the stability assisting apparatus provides a stability assistant function configured to prevent stability-related injuries in real time.

24. The stability assisting apparatus of claim 10, wherein the illumination guidance comprises illuminating a path in a first direction.

25. The stability assisting apparatus of claim 10, wherein the vibration module is configured to provide a haptic feedback through an ankle bone of the user.

26. The stability assisting apparatus of claim 10, wherein the vibration module is configured such that the stability assistant function comprises proprioception feedback such that the stability assistance provides a body location feedback to the user.

27. The stability assisting apparatus of claim 23, wherein the actuator comprises a vibration module (190a) configured to generate vibration stability assistance.

28. The stability assisting apparatus of claim 23, wherein the gait assistance comprises at least one of: haptic, auditory, visual, and/or electrical stimulus.

29. The stability assisting apparatus of claim 23, wherein the gait assistance comprises a warning.

30. The stability assisting apparatus of claim 23, wherein the wearable vibration device is coupled to a body of the user by the helmet.

31. The stability assisting apparatus of claim 23, further comprising a second wearable vibration device configured to be disposed on another location of a body of the user from the helmet.

32. The stability assisting apparatus of claim 23 configured to be a pre-injury detection system.

33. The stability assisting apparatus of claim 23, wherein the sensor module comprises a sensor configured to detect a relative position of the wearable vibration device to the user during limb movements.

## Patentansprüche

1. Eine Gangunterstützungsvorrichtung, umfassend:
ein Kopplungsmodul (116), das zum Koppeln mit einem Benutzer konfiguriert ist; und
eine tragbare Vibrationsvorrichtung (104), die umfasst:
ein Gehäuse (195), das mit dem Kopplungsmodul gekoppelt ist;
ein Sensormodul (1035), das so konfiguriert ist, dass es eine Sensormessung aus gemessenen Daten erzeugt, die mit dem Benutzer in Verbindung stehen;
einen Aktuator (150), der so konfiguriert ist, dass er dem Benutzer eine Gangunterstützung bietet, wobei der Aktuator ein Vibrationsmodul (190a), das so konfiguriert ist, dass es eine Vibrationsgangunterstützung erzeugt, und ein Sichtunterstützungsmodul (190b) umfasst, das so konfiguriert ist, dass es eine Beleuchtungsführung erzeugt;
eine Steuereinheit (1005a), die funktionsfähig mit dem Sensormodul gekoppelt ist und ein Aktivierungsmodul und ein lokales Lernmodell (1020) umfasst, wobei das lokale Lernmodell eine Vielzahl von Gewichtungen umfasst, die so konfiguriert sind, dass sie eine Gangart-Situation auf der Grundlage einer Klassifizierungseingabe klassifizieren, wobei eine Gangart-Situationsklassifizierung keine Gangunterstützung und mindestens eine Intensitätsstufe der Gangunterstützung umfasst, und das Aktivierungsmodul (1025) so konfiguriert ist, dass es einen Aktivierungsgrad erzeugt, um den Aktuator auf der Grundlage der Gangsituationsklassifizierung zu steuern; und
ein Kommunikationsmodul (175), das funktionsfähig mit der Steuerung gekoppelt ist, wobei das Kommunikationsmodul so konfiguriert ist, dass es eine Verbindung zu einem entfernten föderierten maschinellen Lernmodell (1015) herstellt, wobei das entfernte föderierte maschinelle Lernmodell in Datenkommunikation mit einem Netzwerk von entfernten Gangunterstützungsvorrichtungen (1005) verbunden ist, so dass
in einem Online-Modus das lokale Lernmodell so konfiguriert ist, dass es eine Aktualisierungsgewichtungseingabe vom entfernten föderierten maschinellen Lernmodells als Funktion von Trainingseingaben empfängt, wobei die Trainingseingaben aktualisierte Trainingsausgabeparameter umfassen, die von den lokalen Lernmodellen aus dem Netzwerk von entfernten Gangunterstützungsvorrichtungen auf der Grundlage von Sensormessungen von entsprechenden Gangunterstützungsvorrichtungen erzeugt werden; und
in einem Offline-Modus das Aktivierungsmodul nach Empfang der Sensormessung vom Sensormodul das lokale Lernmodell unabhängig auf die Klassifizierungseingabe anwendet, die die empfangene Sensormessung umfasst, um den Aktivierungsgrad des Aktuators so zu bestimmen, dass die Gangunterstützungsvorrichtung eine Gangunterstützungsfunktion bereitstellt, um Gangbeeinträchtigungsverletzungen in Echtzeit zu verhindern.

2. Die Gangunterstützungsvorrichtung nach Anspruch 1, wobei das Kopplungsmodul ein medizinisches Silikonarmband umfasst.

3. Die Gangunterstützungsvorrichtung nach Anspruch 1, wobei das Kopplungsmodul einen Einweggurt umfasst, der am Gehäuse der tragbaren Vibrationsvorrichtung befestigt wird.

4. Die Gangunterstützungsvorrichtung nach Anspruch 1, wobei das Kopplungsmodul für eine untere Extremität des Benutzers konfiguriert ist.

5. Die Gangunterstützungsvorrichtung nach Anspruch 1, wobei das Gehäuse lösbar mit dem Kopplungsmodul verbunden ist.

6. Die Gangunterstützungsvorrichtung nach Anspruch 1, wobei das Sensormodul einen Kraftsensor an einem Innenumfang des Gehäuses umfasst, wobei der Kraftsensor so konfiguriert ist, dass er eine relative Position des tragbaren Vibrationsgeräts zum Benutzer während der Bewegungen der Gliedmaßen erfasst.

7. Die Gangunterstützungsvorrichtung nach Anspruch 1, wobei der Aktuator ferner ein Audiomodul umfasst, das so konfiguriert ist, dass es dem Benutzer akustische Anweisungen gibt.

8. Die Gangunterstützungsvorrichtung nach Anspruch 1, wobei die Aktivierungsstufe eine Vielzahl von Intensitätsstufen der Gangunterstützung umfasst.

9. Die Gangunterstützungsvorrichtung nach Anspruch 1, wobei die Klassifizierungseingabe ferner eine aktuelle Zeit und eine Benutzereingabe umfasst.

10. Eine Stabilitätsunterstützungsvorrichtung, die Folgendes umfasst:
ein Kopplungsmodul (116), das so konfiguriert ist, dass es mit einem Benutzer gekoppelt wird; und
eine tragbare Vibrationsvorrichtung (104), die umfasst:
ein Gehäuse (195), das mit dem Kopplungsmodul gekoppelt ist;
ein Sensormodul (1035), das so konfiguriert ist, dass es eine Sensormessung aus gemessenen Daten erzeugt, die mit dem Benutzer in Verbindung stehen;
einen Aktuator (150), der so konfiguriert ist, dass er dem Benutzer Stabilitätsunterstützung bietet, wobei der Aktuator ein Vibrationsmodul (190a), das so konfiguriert ist, dass es Vibrationsstabilitätsunterstützung erzeugt, und ein Sichtunterstützungsmodul (190b) umfasst, das so konfiguriert ist, dass es eine Beleuchtungsführung erzeugt; und
eine Steuereinheit (1401), die funktionsfähig mit dem Sensormodul gekoppelt ist und ein Aktivierungsmodul (1025) und ein lokales Klassifizierungsmodell (1405) umfasst, wobei das lokale Klassifizierungsmodell eine Vielzahl von Gewichtungen umfasst, die so konfiguriert sind, dass sie eine Gangart-Situation auf der Grundlage einer Klassifizierungseingabe (1430) klassifizieren, wobei die Gangart-Situation keine Gangartunterstützung und mindestens eine Intensitätsstufe der Gangartunterstützung umfasst, und das Aktivierungsmodul so konfiguriert ist, dass es einen Aktivierungsgrad zum Steuern des Aktuators auf der Grundlage der Gangart-Situationsklassifizierung erzeugt, wobei:
im Betrieb das Aktivierungsmodul das lokale Klassifizierungsmodell auf die Klassifizierungseingabe anwendet, die empfangene Sensormessungen umfasst, um die Aktivierungsstufe des Aktuators so zu bestimmen, dass die Stabilitätsunterstützungsvorrichtung eine Stabilitätsunterstützungsfunktion bereitstellt, um stabilitätsbedingte Verletzungen in Echtzeit zu verhindern.

11. Die Stabilitätsunterstützungsvorrichtung nach Anspruch 10, wobei die Stabilitätsunterstützung für den Benutzer eine Gangunterstützung umfasst, die Stabilitätsunterstützungsfunktion eine Gangunterstützungsfunktion umfasst und die stabilitätsbezogenen Verletzungen Gangverletzungen umfassen.

12. Die Stabilitätsunterstützungsvorrichtung nach Anspruch 10, wobei das lokale Klassifizierungsmodell ein in den Controller vorinstalliertes maschinelles Lernmodell umfasst, wobei das maschinelle Lernmodell mit einem gemeinsamen Datensatz vortrainiert ist.

13. Die Stabilitätsunterstützungsvorrichtung nach Anspruch 10, die ferner ein Kommunikationsmodul umfasst, das funktionsfähig mit der Steuereinheit gekoppelt ist, wobei das Kommunikationsmodul so konfiguriert ist, dass es bei Empfang neuer Gewichtungen des lokalen Klassifizierungsmodells das lokale Klassifizierungsmodell aktualisiert.

14. Die Stabilitätsunterstützungsvorrichtung nach Anspruch 10, wobei das Kopplungsmodul ein Band umfasst, das an einem Gliedmaß des Benutzers konfiguriert ist.

15. Die Stabilitätsunterstützungsvorrichtung nach Anspruch 10, wobei das Kopplungsmodul einen Helm umfasst.

16. Die Stabilitätsunterstützungsvorrichtung nach Anspruch 10, wobei das Gehäuse lösbar mit dem Kopplungsmodul gekoppelt ist.

17. Stabilitätsunterstützungsvorrichtung nach Anspruch 10, wobei das Sensormodul einen Kraftsensor an einem Innenumfang des Gehäuses umfasst, wobei der Kraftsensor so konfiguriert ist, dass er eine relative Position der tragbaren Vibrationsvorrichtung zum Benutzer während Gliedmaßenbewegungen erfasst.

18. Eine Gangunterstützungsvorrichtung, die Folgendes umfasst:
ein Kopplungsmodul (116), das so konfiguriert ist, dass es mit einem Benutzer gekoppelt werden kann; und
ein tragbares Vibrationsgerät (104), umfassend:
ein Gehäuse (195), das mit dem Kopplungsmodul gekoppelt ist;
ein Sensormodul (1035), das so konfiguriert ist, dass es eine Sensormessung aus gemessenen Daten erzeugt, die mit dem Benutzer in Verbindung stehen;
einen Aktuator (150), der ein Vibrationsmodul (190a) umfasst, das so konfiguriert ist, dass es eine Vibrationsgangunterstützung erzeugt, um eine Gangunterstützung bereitzustellen;
eine Steuereinheit (1005a), die funktionsfähig mit dem Sensormodul gekoppelt ist und ein Aktivierungsmodul (1020) und ein lokales Lernmodell (1025) umfasst, wobei das lokale Lernmodell eine Vielzahl von Gewichtungen umfasst, die so konfiguriert sind, dass sie eine Gangart-Situation auf der Grundlage einer Klassifizierungseingabe klassifizieren, wobei die Gangart-Situation keine Gangunterstützung und mindestens eine Intensitätsstufe der Gangunterstützung umfasst, und das Aktivierungsmodul so konfiguriert ist, dass es einen Aktivierungsgrad erzeugt, um den Aktuator auf der Grundlage der Gangklassifizierung zu steuern, wobei:
ein Kommunikationsmodul (175), das funktionsfähig mit der Steuerung gekoppelt ist, wobei das Kommunikationsmodul so konfiguriert ist, dass es eine Verbindung zu einem entfernten föderierten maschinellen Lernmodell (1015) herstellt, wobei das entfernte föderierte maschinelle Lernmodell in Datenkommunikation mit einem Netzwerk von entfernten Gangunterstützungsvorrichtungen (1005) verbunden ist, so dass
in einem Online-Modus das lokale Lernmodell so konfiguriert ist, dass es eine Aktualisierungsgewichtungseingabe von dem entfernten föderierten maschinellen Lernmodell als Funktion von Trainingseingaben empfängt, wobei die Trainingseingaben aktualisierte Trainingsausgabeparameter umfassen, die von den lokalen Lernmodellen aus dem Netzwerk von entfernten Gangunterstützungsvorrichtungen auf der Grundlage von Sensormessungen von entsprechenden Gangunterstützungsvorrichtungen erzeugt werden; und
in einem Offline-Modus das Aktivierungsmodul nach Empfang der Sensormessung vom Sensormodul das lokale Lernmodell unabhängig auf die Klassifizierungseingabe anwendet, die die empfangene Sensormessung umfasst, um den Aktivierungsgrad des Aktuators so zu bestimmen, dass die Gangunterstützungsvorrichtung eine Gangunterstützungsfunktion bereitstellt, um Gangbeeinträchtigungsverletzungen in Echtzeit zu verhindern.

19. Die Gangunterstützungsvorrichtung nach Anspruch 18, wobei der Aktuator ferner ein Sichtunterstützungsmodul umfasst, das so konfiguriert ist, dass es eine Beleuchtungsführung erzeugt.

20. Die Gangunterstützungsvorrichtung nach Anspruch 18, wobei das Kopplungsmodul einen Einweggurt umfasst, der am Gehäuse der tragbaren Vibrationsvorrichtung befestigt wird.

21. Die Gangunterstützungsvorrichtung nach Anspruch 18, wobei das Gehäuse lösbar mit dem Kopplungsmodul verbunden ist.

22. Die Gangunterstützungsvorrichtung nach Anspruch 18, wobei das Sensormodul einen Kraftsensor an einem Innenumfang des Gehäuses umfasst, wobei der Kraftsensor so konfiguriert ist, dass er eine relative Position der tragbaren Vibrationsvorrichtung zum Benutzer während Bewegungen der Gliedmaßen erfasst.

23. Eine Stabilitätsunterstützungsvorrichtung, umfassend:
einen Helm (103), der so konfiguriert ist, dass er mit einem Benutzer gekoppelt werden kann; und
ein tragbares Vibrationsgerät (104), umfassend:
ein mit dem Helm verbundenes Gehäuse (195);
ein Sensormodul (1035), das mit dem Helm verbunden ist und so konfiguriert ist, dass es eine Sensormessung aus gemessenen Daten erzeugt, die mit dem Benutzer in Verbindung stehen;
einen Aktuator (150), der mit dem Helm verbunden ist und so konfiguriert ist, dass er dem Benutzer Stabilitätsunterstützung bietet;
eine Steuereinheit (1401), die mit dem Helm verbunden und funktionsfähig mit dem Sensormodul verbunden ist und ein Aktivierungsmodul (1025) und ein lokales Klassifizierungsmodell (1405) umfasst, wobei das lokale Klassifizierungsmodell eine Vielzahl von Gewichtungen umfasst, die so konfiguriert sind, dass sie eine Gangart-Situation auf der Grundlage einer Klassifizierungseingabe (1430) klassifizieren, wobei eine Gangart-Situation-Klassifizierung keine Gangartunterstützung und mindestens eine Intensitätsstufe der Gangartunterstützung umfasst, und das Aktivierungsmodul so konfiguriert ist, dass es einen Aktivierungsgrad zum Steuern des Aktuators auf der Grundlage der Gangart-Situationsklassifizierung erzeugt, so dass dem Benutzer rechtzeitig Unterstützung bereitgestellt wird, wenn Probleme mit der Gangart vorhergesagt werden, wobei:
im Betrieb das Aktivierungsmodul das lokale Klassifizierungsmodell auf die Klassifizierungseingabe anwendet, die empfangene Sensormessungen umfasst, um die Aktivierungsstufe des Aktuators so zu bestimmen, dass die Stabilitätsunterstützungsvorrichtung eine Stabilitätsunterstützungsfunktion bereitstellt, die so konfiguriert ist, dass sie stabilitätsbedingte Verletzungen in Echtzeit verhindert.

24. Die Stabilitätsunterstützungsvorrichtung nach Anspruch 10, wobei die Beleuchtungsführung das Beleuchten eines Weges in einer ersten Richtung umfasst.

25. Die Stabilitätsunterstützungsvorrichtung nach Anspruch 10, wobei das Vibrationsmodul so konfiguriert ist, dass es ein haptisches Feedback über einen Knöchelknochen des Benutzers bereitstellt.

26. Die Stabilitätsunterstützungsvorrichtung nach Anspruch 10, wobei das Vibrationsmodul so konfiguriert ist, dass die Stabilitätsassistenzfunktion ein propriozeptives Feedback umfasst, sodass die Stabilitätsunterstützung dem Benutzer ein Feedback zur Körperposition liefert.

27. Stabilitätsunterstützungsvorrichtung nach Anspruch 23, wobei der Aktuator ein Vibrationsmodul (190a) umfasst, das so konfiguriert ist, dass es eine Vibrationsstabilitätsunterstützung erzeugt.

28. Stabilitätsunterstützungsvorrichtung nach Anspruch 23, wobei die Gangunterstützung mindestens einen der folgenden Stimuli umfasst: haptischen, auditiven, visuellen und/oder elektrischen Stimulus.

29. Die Stabilitätsunterstützungsvorrichtung nach Anspruch 23, wobei die Gangunterstützung eine Warnung umfasst.

30. Die Stabilitätsunterstützungsvorrichtung nach Anspruch 23, wobei das tragbare Vibrationsgerät über den Helm mit dem Körper des Benutzers verbunden ist.

31. Die Stabilitätsunterstützungsvorrichtung nach Anspruch 23, die ferner eine zweite tragbare Vibrationsvorrichtung umfasst, die so konfiguriert ist, dass sie an einer anderen Stelle des Körpers des Benutzers als dem Helm angeordnet werden kann.

32. Die Stabilitätsunterstützungsvorrichtung nach Anspruch 23, die als System zur Erkennung von Verletzungen vor deren Entstehung konfiguriert ist.

33. Stabilitätsunterstützungsvorrichtung nach Anspruch 23, wobei das Sensormodul einen Sensor umfasst, der so konfiguriert ist, dass er eine relative Position des tragbaren Vibrationsgeräts zum Benutzer während Bewegungen der Gliedmaßen erkennt.

## Revendications

1. Appareil d'assistance à la marche comprenant:
un module de couplage (116) configuré pour se coupler à l'utilisateur; et
un dispositif de vibration portable (104) comprenant:
un boîtier (195) couplé au module de couplage;
un module de capteur (1035) configuré pour générer une mesure du capteur à partir des données mesurées associées à l'utilisateur;
un actionneur (150) configuré pour fournir une assistance à la marche à l'utilisateur, l'actionneur comprenant un module de vibration (190a) configuré pour générer une assistance vibratoire à la marche, et un module d'assistance visuelle (190b) configuré pour générer un guidage lumineux;
un contrôleur (1005a) couplé de manière fonctionnelle au module de capteur, comprenant un module d'activation et un modèle d'apprentissage local (1020), le modèle d'apprentissage local comprenant une pluralité de pondérations configurées pour classer une situation de marche sur la base d'une entrée de classification, une classification de la situation de marche ne comprenant aucune assistance à la marche et comprenant au moins un niveau d'intensité de l'assistance à la marche, et le module d'activation (1025) étant configuré pour générer un niveau d'activation pour contrôler l'actionneur sur la base de la classification de la situation de marche; et
un module de communication (175) couplé de manière fonctionnelle au contrôleur, le module de communication étant configuré pour se connecter à un modèle d'apprentissage automatique fédéré distant (1015), le modèle d'apprentissage automatique fédéré distant étant relié à un réseau d'appareils d'assistance à la marche distants (1005) dans une communication de données de telle sorte que,
dans un mode en ligne, le modèle d'apprentissage local est configuré pour recevoir une entrée de la mise à jour des pondérations du modèle d'apprentissage automatique fédéré distant en fonction des entrées d'entraînement, les entrées d'entraînement comprenant des paramètres de sortie d'entraînement mis à jour, générés par les modèles d'apprentissage locaux du réseau d'appareils d'assistance à la marche distants sur la base de mesures du capteur des appareils d'assistance à la marche correspondants; et
dans un mode hors ligne, à la réception de la mesure du capteur provenant du module de capteur, le module d'activation applique indépendamment le modèle d'apprentissage local à l'entrée de classification comprenant la mesure du capteur reçue afin de déterminer le niveau d'activation de l'actionneur de telle sorte que l'appareil d'assistance à la marche fournit une fonction d'assistant à la marche pour prévenir les blessures dues aux troubles de la marche en temps réel.

2. Appareil d'assistance à la marche selon la revendication 1, dans lequel le module de couplage comprend un bracelet en silicone de qualité médicale.

3. Appareil d'assistance à la marche selon la revendication 1, dans lequel le module de couplage comprend une sangle disponible qui se fixe au boîtier du dispositif de vibration portable.

4. Appareil d'assistance à la marche selon la revendication 1, dans lequel le module de couplage est configuré pour un membre inférieur de l'utilisateur.

5. Appareil d'assistance à la marche selon la revendication 1, dans lequel le boîtier est couplé de manière amovible au module de couplage.

6. Appareil d'assistance à la marche selon la revendication 1, dans lequel le module de capteur comprend un capteur de force situé sur un périmètre intérieur du boîtier, le capteur de force étant configuré pour détecter une position relative du dispositif de vibration portable par rapport à l'utilisateur lors des mouvements des membres.

7. Appareil d'assistance à la marche selon la revendication 1, dans lequel l'actionneur comprend en outre un module audio configuré pour générer un guidage sonore à l'utilisateur.

8. Appareil d'assistance à la marche selon la revendication 1, dans lequel le niveau d'activation comprend une pluralité de niveaux d'intensité de l'assistance à la marche.

9. Appareil d'assistance à la marche selon la revendication 1, dans lequel l'entrée de classification comprend en outre l'heure actuelle et une entrée d'utilisateur.

10. Appareil d'assistance à la stabilité comprenant:
un module de couplage (116) configuré pour se coupler à l'utilisateur; et
un dispositif de vibration portable (104) comprenant:
un boîtier (195) couplé au module de couplage;
un module de capteur (1035) configuré pour générer une mesure du capteur à partir des données mesurées associées à l'utilisateur;
un actionneur (150) configuré pour fournir une assistance à la stabilité à l'utilisateur, l'actionneur comprenant un module de vibration (190a) configuré pour générer une assistance vibratoire à la stabilité, et un module d'assistance visuelle (190b) configuré pour générer un guidage lumineux; et
un contrôleur (1401) couplé de manière fonctionnelle au module de capteur, comprenant un module d'activation (1025) et un modèle de classification local (1405), le modèle de classification local comprenant une pluralité de pondérations configurées pour classer une situation de marche sur la base d'une entrée de classification (1430), la situation de marche ne comprenant aucune assistance à la marche et comprenant au moins un niveau d'intensité de l'assistance à la marche, et le module d'activation étant configuré pour générer un niveau d'activation pour contrôler l'actionneur sur la base de la classification de la situation de marche, dans lequel,
en fonctionnement, le module d'activation applique le modèle de classification local à l'entrée de classification comprenant des mesures de capteur reçues pour déterminer le niveau d'activation de l'actionneur de telle sorte que l'appareil d'assistance à la stabilité fournit une fonction d'assistant à la stabilité pour prévenir les blessures liées à la stabilité en temps réel.

11. Appareil d'assistance à la stabilité selon la revendication 10, dans lequel l'assistance à la stabilité fournie à l'utilisateur comprend une assistance à la marche, la fonction d'assistant à la stabilité comprend une fonction d'assistant à la marche et les blessures liées à la stabilité comprennent des blessures dues à la marche.

12. Appareil d'assistance à la stabilité selon la revendication 10, dans lequel le modèle de classification local comprend un modèle d'apprentissage automatique préchargé dans le contrôleur, le modèle d'apprentissage automatique étant pré-entraîné à l'aide d'un ensemble commun de données.

13. Appareil d'assistance à la stabilité selon la revendication 10, comprenant en outre un module de communication couplé de manière fonctionnelle au contrôleur, le module de communication étant configuré pour mettre à jour le modèle de classification local à la réception de nouvelles pondérations du modèle de classification local.

14. Appareil d'assistance à la stabilité selon la revendication 10, dans lequel le module de couplage comprend une bande configurée pour un membre de l'utilisateur.

15. Appareil d'assistance à la stabilité selon la revendication 10, dans lequel le module de couplage comprend un casque.

16. Appareil d'assistance à la stabilité selon la revendication 10, dans lequel le boîtier est couplé de manière amovible au module de couplage.

17. Appareil d'assistance à la stabilité selon la revendication 10, dans lequel le module de capteur comprend un capteur de force situé sur un périmètre intérieur du boîtier, le capteur de force étant configuré pour détecter une position relative du dispositif de vibration portable par rapport à l'utilisateur lors des mouvements des membres.

18. Appareil d'assistance à la marche comprenant:
un module de couplage (116) configuré pour se coupler à l'utilisateur; et
un dispositif de vibration portable (104) comprenant:
un boîtier (195) couplé au module de couplage;
un module de capteur (1035) configuré pour générer une mesure du capteur à partir des données mesurées associées à l'utilisateur;
un actionneur (150) comprenant un module de vibration (190a) configuré pour générer une assistance vibratoire à la marche pour fournir une assistance à la marche;
un contrôleur (1005a) couplé de manière fonctionnelle au module de capteur, comprenant un module d'activation (1020) et un modèle d'apprentissage local (1025), le modèle d'apprentissage local comprenant une pluralité de pondérations configurées pour classer une situation de marche sur la base d'une entrée de classification, la situation de marche ne comprenant aucune assistance à la marche et comprenant au moins un niveau d'intensité de l'assistance à la marche, et le module d'activation étant configuré pour générer un niveau d'activation pour contrôler l'actionneur sur la base de la classification de la marche, dans lequel:
un module de communication (175) couplé de manière fonctionnelle au contrôleur, le module de communication étant configuré pour se connecter à un modèle d'apprentissage automatique fédéré distant (1015), le modèle d'apprentissage automatique fédéré distant étant relié à un réseau d'appareils d'assistance à la marche distants (1005) dans une communication de données de telle sorte que,
dans un mode en ligne, le modèle d'apprentissage local est configuré pour recevoir une entrée de la mise à jour des pondérations du modèle d'apprentissage automatique fédéré distant en fonction des entrées d'entraînement, les entrées d'entraînement comprenant des paramètres de sortie d'entraînement mis à jour, générés par les modèles d'apprentissage locaux du réseau d'appareils d'assistance à la marche distants sur la base de mesures du capteur des appareils d'assistance à la marche correspondants; et
dans un mode hors ligne, à la réception de la mesure du capteur provenant du module de capteur, le module d'activation applique indépendamment le modèle d'apprentissage local à l'entrée de classification comprenant la mesure du capteur reçue afin de déterminer le niveau d'activation de l'actionneur de telle sorte que l'appareil d'assistance à la marche fournit une fonction d'assistant à la marche pour prévenir les blessures dues aux troubles de la marche en temps réel.

19. Appareil d'assistance à la marche selon la revendication 18, dans lequel l'actionneur comprend en outre un module d'assistance visuelle configuré pour générer un guidage lumineux.

20. Appareil d'assistance à la marche selon la revendication 18, dans lequel le module de couplage comprend une sangle disponible qui se fixe au boîtier du dispositif de vibration portable.

21. Appareil d'assistance à la marche selon la revendication 18, dans lequel le boîtier est couplé de manière amovible au module de couplage.

22. Appareil d'assistance à la marche selon la revendication 18, dans lequel le module de capteur comprend un capteur de force situé sur un périmètre intérieur du boîtier, le capteur de force étant configuré pour détecter une position relative du dispositif de vibration portable par rapport à l'utilisateur lors des mouvements des membres.

23. Appareil d'assistance à la stabilité comprenant:
un casque (103) configuré pour se coupler à l'utilisateur; et
un dispositif de vibration portable (104) comprenant:
un boîtier (195) couplé au casque;
un module de capteur (1035) couplé au casque et configuré pour générer une mesure du capteur à partir des données mesurées associées à l'utilisateur;
un actionneur (150) couplé au casque et configuré pour fournir une assistance à la stabilité à l'utilisateur;
un contrôleur (1401) couplé au casque et couplé de manière fonctionnelle au module de capteur, comprenant un module d'activation (1025) et un modèle de classification local (1405), le modèle de classification local comprenant une pluralité de pondérations configurées pour classer une situation de marche sur la base d'une entrée de classification (1430), une classification de la situation de marche ne comprenant aucune assistance à la marche et comprenant au moins un niveau d'intensité de l'assistance à la marche, et le module d'activation étant configuré pour générer un niveau d'activation pour contrôler l'actionneur sur la base de la classification de la situation de marche de telle sorte qu'une assistance à temps est fournie sélectivement à l'utilisateur lorsqu'il est prévu qu'il ait des difficultés de marche, dans lequel:
en fonctionnement, le module d'activation applique le modèle de classification local à l'entrée de classification comprenant des mesures de capteur reçues pour déterminer le niveau d'activation de l'actionneur de telle sorte que l'appareil d'assistance à la stabilité fournit une fonction d'assistant à la stabilité configurée pour prévenir les blessures liées à la stabilité en temps réel.

24. Appareil d'assistance à la stabilité selon la revendication 10, dans lequel le guidage lumineux comprend illuminer un chemin dans une première direction.

25. Appareil d'assistance à la stabilité selon la revendication 10, dans lequel le module de vibration est configuré pour fournir un retour haptique via un os de la cheville de l'utilisateur.

26. Appareil d'assistance à la stabilité selon la revendication 10, dans lequel le module de vibration est configuré de telle sorte que la fonction d'assistant à la stabilité comprend un retour proprioceptif de manière que l'assistance à la stabilité fournisse un retour sur la position du corps à l'utilisateur.

27. Appareil d'assistance à la stabilité selon la revendication 23, dans lequel l'actionneur comprend un module de vibration (190a) configuré pour générer une assistance vibratoire à la stabilité.

28. Appareil d'assistance à la stabilité selon la revendication 23, dans lequel l'assistance à la marche comprend au moins un des stimuli suivants: un stimulus haptique, un stimulus auditif, un stimulus visuel et/ou un stimulus électrique.

29. Appareil d'assistance à la stabilité selon la revendication 23, dans lequel l'assistance à la marche comprend une alerte.

30. Appareil d'assistance à la stabilité selon la revendication 23, dans lequel le dispositif de vibration portable est couplé au corps de l'utilisateur par le casque.

31. Appareil d'assistance à la stabilité selon la revendication 23, comprenant en outre un deuxième dispositif de vibration portable configuré pour être disposé à un autre endroit du corps de l'utilisateur, par rapport au casque.

32. Appareil d'assistance à la stabilité selon la revendication 23, configuré pour être un système de détection préventive des blessures.

33. Appareil d'assistance à la stabilité selon la revendication 23, dans lequel le module de capteur comprend un capteur configuré pour détecter une position relative du dispositif de vibration portable par rapport à l'utilisateur lors des mouvements des membres.
